(19) Europäisches Patentamt
European Patent Office
Office européen des brevets

(11) Veröffentlichungsnummer: **0 041 215**
**B1**

# (12) EUROPÄISCHE PATENTSCHRIFT

(45) Veröffentlichungstag der Patentschrift: **29.01.86**

(21) Anmeldenummer: **81104000.5**

(22) Anmeldetag: **25.05.81**

(51) Int. Cl.⁴: **C 07 D 513/04**, C 07 D 498/04, C 07 D 487/04, A 61 K 31/42, A 61 K 31/415, A 61 K 31/425 // (C07D513/04, 285:00, 235:00),(C07D513/04, 277:00, 235:00),(C07D498/04, 271:00, 235:00),(C07D487/04, 249:00, 235:00),(C07D498/04, 263:00, 235:00),(C07D487/04, 235:00, 235:00)

(54) **Imidazoazolalkensäureamide, neue Zwischenprodukte zu ihrer Herstellung, ihre Herstellung und ihre Verwendung in Arzneimitteln.**

(30) Priorität: **29.05.80 DE 3020421**
**15.11.80 DE 3043158**

(43) Veröffentlichungstag der Anmeldung: **09.12.81 Patentblatt 81/49**

(45) Bekanntmachung des Hinweises auf die Patenterteilung: **29.01.86 Patentblatt 86/5**

(84) Benannte Vertragsstaaten: **AT BE CH DE FR GB IT LI LU NL SE**

(56) Entgegenhaltungen:
**AT - A - 340 414**
**US - A - 3 615 639**

**Eur. J. Med. Chem., Jan-Febr. 1975, S. 59-64**
**J. Het. Chem., Sept. 79, S. 1201-1207**
**Il Farmaco, Ed. Sc. 35 (1979/80), S. 573-580**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Meyer, Horst, Dr., Theodor-Heuss-Strasse 110, D-5600 Wuppertal 1 (DE)**
Erfinder: **Horstmann, Harald, Dr., Claudiusweg 19, D-5600 Wuppertal 1 (DE)**
Erfinder: **Möller, Eike, Dr., Pahlkestrasse 37, D-5600 Wuppertal 1 (DE)**
Erfinder: **Garthoff, Bernward, Dr., Händelstrasse 22, D-4010 Hilden (DE)**

**EP 0 041 215 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Imidazoazolalkensäureamide, mehrere Verfahren zu ihrer Herstellung, neue Zwischenprodukte, die bei ihrer Herstellung eingesetzt werden, und die Verwendung von Imidazo-alkensäureamiden in Arzneimitteln, insbesondere in Antihypertensiva, Diuretika und Uricosurica.

Es ist bereits bekannt geworden, dass bestimmte Imidazothiadiazole biologische Wirkungen, insbesondere antithrombotische und antimikrobielle Eigenschaften besitzen (vgl. DE-OS 2 823 682).

Weiterhin ist bekannt, dass bestimmte Imidazothiadiazole und Imidazo-thiazolsulfonamide zerebrale Wirkungen besitzen (J. med. Chem. 1980, 117, I.C. Barnisch et al.).

Aus AT-A 340 414 und J.F. Robert et al., J. Heterocycl. Chem. 16, 1201 (1979) ist bekannt, dass Imidazo-thiazole und -thiadiazole antidiabetische Wirkungen aufweisen. Verbindungen dieser Art werden auch von K.C. Joshi et al. in J. Indian Chem. Soc. 56, 716 (1979) und M.A. Eldaway et al. in Pharmazie 34, 144 (1979) beschrieben.

Dem Stand der Technik sind jedoch nirgendwo Imidazo-thiadiazole und Imidazo-thiazole zu entnehmen, welche die erfindungswesentliche Seitengruppe in 5-Position aufweisen. Wie sich zeigte, sind die neuen Imidazoazoalkensäureamide durch ihre diuretische und uricosurische Wirkung ausgezeichnet.

Gegenstand der Erfindung sind Imidazolalkensäureamide der allgemeinen Formel

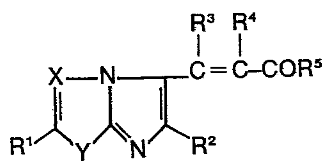

(I)

in welcher

X für N oder CH steht,

Y für S, O, NH oder N-Alkyl mit 1 bis 4 Kohlenstoffatomen steht,

$R^1$ und $R^2$ gleich oder verschieden sind und jeweils für Wasserstoff, geradkettiges oder verzweigtes oder cyclisches Alkyl, Alkenyl oder Alkinyl mit bis zu 6 Kohlenstoffatomen, welches gegebenenfalls substituiert ist durch Phenyl, Cyano, Hydroxyl, Methyl, Ethyl, Methoxy, Ethoxy, Trifluormethyl, Fluor, Chlor oder Brom stehen, wobei die Kohlenstoffkette des Alkylrestes gegebenenfalls unterbrochen ist durch O, S, NH, N-Alkyl mit 1 bis 4 Kohlenstoffatomen oder durch N-Benzyl,

oder

für einen Phenyl-, Naphthyl-, Furyl-, Thienyl-, Pyrimidyl-, Pyrazinyl-, Chinolyl-, Isochinolyl- oder Pyridylring stehen, der gegebenenfalls substituiert ist durch 1, 2 oder 3 gleiche oder verschiedene Substituenten aus der Gruppe Nitro, Trifluormethyl, Fluor, Chlor, Brom, Alkyl, Alkoxy oder $SO_n$-Alkyl (n = 0, 1 oder 2), wobei die genannten Alkyl- und Alkoxyreste jeweils 1 bis 4 Kohlenstoffatome enthalten,

für eine $SO_n$-Alkylgruppe stehen, wobei n und Alkyl die oben angegebene Bedeutung haben,

oder für den Rest 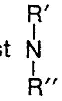

stehen, wobei R′ und R″ gleich oder verschieden sind und jeweils für Wasserstoff, Benzyl, Phenyl oder geradkettiges, verzweigtes oder cyclisches Alkyl mit 1 bis 6 Kohlenstoffatomen stehen, wobei die Alkylreste ihrerseits durch O, S oder N-Alkyl (1–4 C-Atome) unterbrochen sein können oder wobei R′ und R″ gemeinsam mit dem Stickstoffatom einen 5- bis 7-gliedrigen Ring bilden, der seinerseits gegebenenfalls 1 oder 2 weitere gleiche oder verschiedene Heteroatome aus der Gruppe Sauerstoff, Schwefel oder Stickstoff enthalten kann, wobei der Stickstoff gegebenenfalls durch Wasserstoff, Alkyl mit 1 bis 4 Kohlenstoffatomen, Phenyl oder Benzyl substituiert ist,

oder

für den Rest COR‴ stehen, wobei R‴ Wasserstoff, Hydroxyl, Alkyl, Alkoxy mit jeweils 1 bis 6 C-Atomen, Phenoxy, Benzyloxy, Alkenoxy mit bis zu 4 C-Atomen, oder den Rest 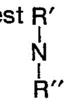

bedeutet, wobei R′ und R″ die oben angegebene Bedeutung haben,

$R^3$ für Wasserstoff, Trifluormethyl oder Alkyl mit 1 bis 4 Kohlenstoffatomen steht,

$R^4$ für Wasserstoff, Cyano, Nitro, Fluor, Chlor, Brom oder $SO_n$-Alkyl (1–4 C-Atome; n = 0, 1 oder 2) steht, oder für Alkoxy mit 1 bis 4 C-Atomen steht,

oder

für geradkettiges, verzweigtes oder cyclisches Alkyl, Alkenyl oder Alkinyl mit jeweils bis zu 6 C-Atomen steht, wobei der Alkylrest gegebenenfalls substituiert ist durch Fluor, Chlor, Brom oder Cyano und gegebenenfalls unterbrochen ist durch O, S, NH, N-Benzyl oder N-Alkyl (1–4 C-Atome) oder für den Rest COR‴ steht, wobei R‴ die oben in der Definition von $R^1$ und $R^2$ angegebene Bedeutung besitzt,

und mit diesem identisch oder verschieden sein kann, und

$R^5$ für eine Aminogruppe der Formel

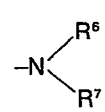

steht,

in welcher

a) $R^6$ Wasserstoff, Phenyl oder einen geradkettigen, verzweigten, cyclischen, gesättigten oder ungesättigten aliphatischen Kohlenwasserstoffrest mit bis zu 10 Kohlenstoffatomen bedeutet, der gegebenenfalls durch O, S, N, N-Alkyl (1–4 C-Atome), NH, N-Phenyl oder N-Benzyl unterbrochen ist und der gegebenenfalls substituiert ist durch Hydroxy, Alkoxy, Alkyl, Fluor, Chlor, Brom, Phenyl, Alkoxycarbonyl oder Dialkylamino mit jeweils 1 bis 4 C-Atomen in den Alkyl- und Alkoxyresten, wobei die beiden Alkylreste gegebenenfalls mit dem N-Atom einen 5- bis 7-gliedrigen Ring bilden, der gegebenenfalls durch O, S, NH oder N-Alkyl (1 bis 4 C-Atome) unterbrochen ist, und wobei die vorgenannten Alkyl- und Phenylreste ihrerseits gegebenenfalls substituiert sind durch Fluor, Chlor, Brom, Trifluormethyl, Phenyl, Benzyl, Alkyl, Alkoxy oder $SO_2$-Alkyl, mit jeweils 1 bis 4 C-Atomen in den Alkyl- und Alkoxyresten oder

b) $R^6$ und $R^7$ gemeinsam mit dem Stockstoffatom einen 4- bis 7-gliedrigen Ring bilden, der gegebenenfalls 1 oder 2 weitere Heteroatome aus der Gruppe Sauerstoff, Schwefel oder Stickstoff enthält, wobei der Stickstoff gegebenenfalls durch Wasserstoff, Alkyl (1–4 C-Atome), Phenyl oder Benzyl substituiert ist und wobei dieser 4- bis 7-gliedrige Ring durch 1 bis 4 gleiche oder verschiedene Substituenten aus der Gruppe Halogen, Trifluormethyl, Phenyl, Benzyl, Alkyl, Hydroxyalkyl, Alkoxyalkyl oder Alkoxycarbonyl mit jeweils 1 bis 4 C-Atomen in den Alkyl- und Alkoxyresten substituiert sein kann, oder wobei dieser Ring mit einem gegebenenfalls durch Fluor, Chlor, Brom, Nitro oder Hydroxy substituierten aromatischen Ring kondensiert sein kann,

c) $R^7$ die unter a) angegebene Bedeutung von $R^6$ besitzt, wobei $R^7$ und $R^6$ gleich oder verschieden sein können,

oder

d) einer der beiden Reste $R^6$ oder $R^7$ für die Gruppe

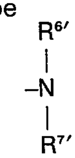
,

steht, wobei die Reste $R^{6'}$ und $R^{7'}$ die unter a) und b) angegebene Bedeutung von $R^6$ und $R^7$ haben (Hydrazine)

und ihre pharmazeutisch unbedenklichen Säureadditionssalze sowie ihre stereoisomeren Formen.

Die Herstellung der erfindungsgemässen Imidazo-alkensäureamide der allgemeinen Formel (I) erfolgt, indem man

a) Carbonylverbindungen der allgemeinen Formel (II)

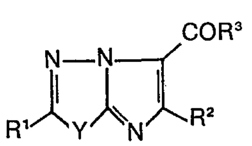
(II)

in welcher

X, Y, $R^1$, $R^2$ und $R^3$ die oben angegebene Bedeutung haben,

mit Phosphonatverbindungen der allgemeinen Formel (III)

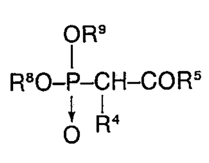
(III)

in welcher

$R^4$ und $R^5$ die oben angegebene Bedeutung haben, und $R^8$ und $R^9$ für gegebenenfalls substituiertes Alkyl oder Aralkyl stehen,

in Gegenwart von starken Basen und inerten organischen Lösungsmitteln bei Temperaturen zwischen $-20\,°C$ und $+40\,°C$ umsetzt oder

b) Carbonylverbindungen der allgemeinen Formel (II) mit Acetamidderivaten der allgemeinen Formel (IV)

$$R^4–CH_2–COR^5 \qquad (IV)$$

in welcher

$R^4$ und $R^5$ die oben angegebene Bedeutung haben,

in Gegenwart von sauren oder basischen Katalysatoren und gegebenenfalls in Gegenwart von inerten organischen Lösungsmitteln bei Temperaturen zwischen 20 und 200 °C umsetzt oder

c) Alkensäuren der allgemeinen Formel (V)

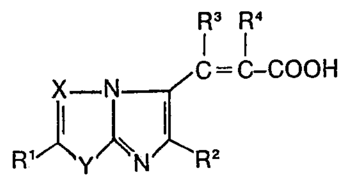
(V)

in welcher

X, Y, $R^1$, $R^2$, $R^3$ und $R^4$ die oben angegebene Bedeutung haben,

mit Aminen der allgemeinen Formel (VI)

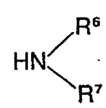
(VI)

in welcher

$R^6$ und $R^7$ die oben angegebene Bedeutung haben,

gegebenenfalls nach Aktivierung der Carboxylgruppe über das entsprechende Säurehalogenid in üblicher Weise, gegebenenfalls in Gegenwart von inerten organischen Lösungsmitteln bei Temperaturen zwischen 20 und 150 °C amidiert.

Je nach Wahl der Ausgangssubstanzen können die erfindungsgemässen Verbindungen in stereoisomeren Formen existieren, die sich entweder wie Bild und Spiegelbild (Enantiomere) oder die sich nicht wie Bild und Spiegelbild (Diastereomere) verhalten. Sowohl die Antipoden als auch die Racemformen als auch die Diastereomerengemische sind Gegenstand der vorliegenden Erfindung. Die Racemformen lassen sich ebenso wie die Diastereomeren in bekannter Weise in die stereoisomer einheitlichen Bestandteile trennen (vgl. z.B. E.L. Eliel, Stereochemistry of Carbon Compounds, McGraw Hill, 1962).

Darüber hinaus werden die durch die Doppelbindung der Seitenkette möglichen Z/E-Isomeren beansprucht, die nach bekannten Verfahren hergestellt oder nach bekannten Verfahren ineinander übergeführt werden können.

Die als Ausgangsverbindungen einsetzbaren Carbonylverbindungen der allgemeinen Formel (II) sind bisher nicht bekannt, können aber in analoger Weise nach folgenden bekannten Methoden hergestellt werden.

Methode a)
L. Pentimalli et al, Boll. Sci. Fac. Chim. Ind. Bologna 23, 181 (1965); s. Chem. Abstr. 63, 17848 e (1965).

Methode b)
D. Bower et al; J. Chem. Soc. 1955, 1834.

Methode c)
A. Hetzheim et al, Chem. Ber. 103, 3533 (1970).

Methode d)
H. Beyer et al, Z. Chem. 2, 152 (1962).

Methode e)
S. Kano, Yakugku Zasski 92, 935 (1972).

Die vorliegende Erfindung betrifft somit auch neue Verbindungen der allgemeinen Formel (II) in welcher X, Y, $R^1$, $R^2$ und $R^3$ die Bedeutungen haben, die für diese Substituenten für Verbindungen der allgemeinen Formel (I) angegeben sind.

Die als Ausgangsverbindungen einsetzbaren Alkensäuren der allgemeinen Formel (V) sind neu. Sie können nach bekannten Methoden hergestellt werden, indem man

a) Carbonylverbindungen der allgemeinen Formel (II)

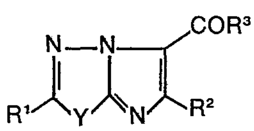

(II)

in welcher
X, Y, $R^1$, $R^2$ und $R^3$ die oben angegebene Bedeutung haben,
mit Phosphonatverbindungen der allgemeinen Formel (VII)

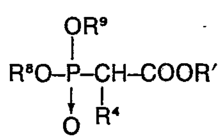

(VII)

in welcher
$R^4$, $R^8$ und $R^9$ die oben angegebene Bedeutung besitzen und
R′ für gegebenenfalls substituiertes Alkyl oder Aralkyl steht,
zu Alkensäureestern umsetzt und diese dann in Gegenwart von Basen zu den entsprechenden Alkensäuren der allgemeinen Formel (V) verseift (vgl. W.S. Wadsworth et al, JACS 83, 1733 (1961)) oder

b) für den Fall, dass $R^3$ Wasserstoff bedeutet, Aldehyde der allgemeinen Formel (VIII)

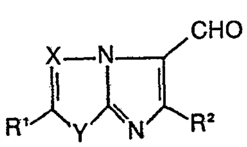

(VIII)

in welcher
X, Y, $R^1$ und $R^2$ die oben angegebene Bedeutung haben, mit Malonsäuren der allgemeinen Formel (IX)

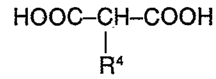

(IX)

in welcher
$R^4$ die oben angegebene Bedeutung hat,
in Gegenwart von inerten organischen Lösungsmitteln und gegebenenfalls in Gegenwart von Kondensationsmitteln kondensiert (vgl. G. Jones, Org. Reactions, Vol. 15, S. 204 ff).

Von besonderem Interesse sind Verbindungen der allgemeinen Formel (I), in welcher
X für N oder CH steht,
Y für S, O oder NH steht,
$R^1$ und $R^2$ gleich oder verschieden sind und jeweils für Wasserstoff, Trifluormethyl, Phenyl, Alkyl oder Alkenyl mit bis zu 6 Kohlenstoffatomen, gegebenenfalls substituiert durch Fluor, Chlor, Hydroxyl, Trifluormethyl, Alkyl oder Alkoxy mit je 1 bis 2 Kohlenstoffatomen, oder für Pyridyl, Thienyl, Furyl, Naphthyl, Pyrimidyl, Pyrazinyl, Chinolyl oder Isochinolyl stehen,
oder
für den Rest 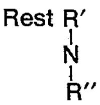
stehen, wobei R′ und R″ gleich oder verschieden sind und jeweils für Wasserstoff, Benzyl, Phenyl oder geradkettiges, verzweigtes oder cyclisches Alkyl mit bis zu 6 Kohlenstoffatomen stehen, wobei die Alkylreste ihrerseits durch O, S oder N-Alkyl (1–2 C-Atome) unterbrochen sein können oder wobei R′ oder R″ gemeinsam mit dem Stickstoffatom einen 5- bis 7-gliedrigen Ring bilden, der seinerseits 1 oder 2 gleiche oder verschiedene Heteroatome aus der Gruppe Sauerstoff, Schwefel oder Stickstoff enthalten kann, wobei der Stickstoff gegebenenfalls durch Wasserstoff, Alkyl mit 1 bis 2 Kohlenstoffatomen, Phenyl oder Benzyl substituiert ist,
oder
für den Rest COR‴ stehen, wobei R‴ Wasserstoff, Hydroxyl, Alkyl, Alkoxy mit jeweils bis zu 4 Kohlenstoffatomen, Phenoxy, Benzyloxy oder den Rest 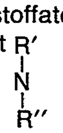
bedeutet, wobei R′ und R″ die oben angegebene Bedeutung haben,
$R^3$ für Wasserstoff, Trifluormethyl oder Alkyl mit 1 bis 4 Kohlenstoffatomen steht,
$R^4$ für Wasserstoff, Alkyl oder Alkoxy mit jeweils 1 bis 4 Kohlenstoffatomen, gegebenenfalls unterbrochen durch Sauerstoff und gegebenenfalls substituiert durch Fluor, Chlor oder Cyano steht oder
für Cyano, Nitro oder für den Rest COR‴ steht, wobei R‴ die oben in der Definition von $R^1$ und $R^2$ angegebene Bedeutung besitzt,

oder

für $SO_n$-Alkyl (n = 0 oder 2) mit 1 bis 4 Kohlenstoffatomen im Alkylteil steht, und

$R^5$ für eine Aminogruppe der Formel

$$-N\begin{matrix} R^{6'} \\ | \\ \\ | \\ R^{7'} \end{matrix} \quad ,$$

steht,

in welcher

a) $R^6$ Wasserstoff, Phenyl oder einen geradkettigen, verzweigten, cyclischen, gesättigten oder ungesättigten Kohlenwasserstoffrest mit bis zu 10 Kohlenstoffatomen bedeutet, der gegebenenfalls durch O, S, N, NH, N-Phenyl oder N-Benzyl unterbrochen ist und der gegebenenfalls substituiert ist durch Hydroxy, Alkoxy, Fluor, Chlor, Brom, Phenyl, Alkoxycarbonyl oder Dialkylamino mit jeweils 1 bis 4 C-Atomen, in den Alkyl- und Alkoxyresten, wobei die beiden Alkylreste gegebenenfalls mit dem N-Atom einen 5- oder 6-gliedrigen Ring bilden, der gegebenenfalls durch O, S oder NH unterbrochen ist, und wobei die vorgenannten Alkyl- und Phenylreste ihrerseits gegebenenfalls substituiert sind durch Fluor, Chlor, Brom, Trifluormethyl, Alkyl, Alkoxy oder $SO_2$-Alkyl, mit jeweils 1 bis 4 C-Atomen in den Alkyl- und Alkoxyresten,

oder

b) $R^6$ gemeinsam mit $R^7$ mit dem Stickstoffatom einen 4- bis 7-gliedrigen Ring bilden, der gegebenenfalls 1 oder 2 weitere Heteroatome aus der Gruppe Sauerstoff, Schwefel oder Stickstoff enthält, wobei der Stickstoff gegebenenfalls durch Wasserstoff, Alkyl (1–2 C-Atome), Phenyl oder Benzyl substituiert ist und wobei dieser 4- bis 7-gliedrige Ring durch 1 bis 4 gleiche oder verschiedene Substituenten aus der Gruppe Fluor, Chlor, Trifluormethyl, Alkyl, Hydroxyalkyl, Alkoxyalkyl oder Alkoxycarbonyl mit jeweils 1 bis 4 C-Atomen in den Alkyl- und Alkoxyresten substituiert sein kann, oder

wobei dieser Ring mit einem gegebenenfalls durch Fluor, Chlor, Nitro oder Hydroxy substituierten aromatischen Ring kondensiert sein kann,

c) $R^7$ die unter a) angegebene Bedeutung von $R^6$ besitzt, wobei $R^7$ und $R^6$ gleich oder verschieden sein können

oder

d) einer der beiden Reste $R^6$ oder $R^7$ für die Gruppe

$$-N\begin{matrix} R^{6'} \\ | \\ \\ | \\ R^{7'} \end{matrix} \quad ,$$

steht, wobei die Reste $R^{6'}$ und $R^{7'}$ die unter a) und b) angegebene Bedeutung von $R^6$ und $R^7$ haben (Hydrazine)

und ihre pharmazeutisch unbedenklichen Säureadditionssalze, sowie ihre stereoisomeren Formen.

Die neuen erfindungsgemässen Verbindungen zeichnen sich überraschenderweise durch starke biologische Wirkungen aus. Insbesondere besitzen sie ausgeprägte diuretische und saluretische Wirkungen und können daher als Diuretika, Saluretika und Antihypertensiva verwendet werden. Bei Tierversuchen an Mäusen, Ratten bzw. Hunden zeigt sich, dass die erfindungsgemässen Verbindungen bei oraler Applikation bereits bei Dosierungen unter 10 mg/kg eine ausgeprägte diuretische und saluretische Wirkung bei gleichzeitiger guter Verträglichkeit besitzen. Bei Kenntnis des Standes der Technik konnten diese vorteilhaften Eigenschaften nicht erwartet werden.

Die überraschenden und vorteilhaften Wirkungen der erfindungsgemässen Verbindungen lassen sich nach den folgenden Testmethoden ermitteln:

A) Antihypertensive Wirkung an Ratten

Die Blutdruckwirkung wird ermittelt an Goldblatthochdruckratten nach Breuninger, H.: Methode zur unblutigen Messung des Blutdruckes an Kleintieren, Arzneimittelforsch. 6, 222–225 (1965).

B) Diuretische Wirkung an Ratten

Nüchterne männliche Ratten im Gewicht von 150 bis 250 g (SPF, Wistar, je n = 4 Paare) werden mit 10 ml/kg p.o. Tylose-Suspension (0,5%) als Kontrollen bzw. mit 100 mg/kg p.o. Prüfsubstanz in 10 ml/kg p.o. Tylose-Suspension mittels Schlundsonde behandelt. Die Tiere werden in Stoffwechselkäfige verbracht und die Harn- und Elektrolytausscheidung über 6 Stunden bestimmt ($Na^+$ und $K^+$ Bestimmung: IL-Flammenphotometer).

C) Diuretische Wirkung an Hunden

Bei nüchternen, wachen weiblichen Beagle-Hunden wird die Harnblase katheterisiert und die Harn- und Elektrolytausscheidung über 180 Minuten (fraktioniert über jeweils 30 Minuten) bestimmt.

Die Tiere erhalten während dieser Zeit über eine Dauerinfusion eine Elektrolytlösung intravenös und am Beginn des Versuches die Prüfsubstanz in 2 ml/kg Tylose-Suspension (0,5%) oral zugeführt.

Der Harn wird auf $Na^+$, $K^+$, Chlor, Bicarbonat und pH analysiert.

D) Diuretische Wirkung an Mäusen

Nüchterne männliche SPF-Mäuse im Gewicht von 20 bis 25 g (n = 6 × 3 Tiere/Käfig) erhalten 100 ml/kg Tylose-Suspension (0,5%) als Kontrollen, bzw. 100 mg/kg Prüfsubstanz in Tylose-Suspension oral zugeführt.

Die Ausscheidung von Harn, $Na^+$ und $K^+$ bzw. Harnsäure wird über 2 und 4 Stunden in Stoffwechselkäfigen bestimmt.

E) Phenolrot-Retentionstest an Ratten

Zum Nachweis von uricosurischer Wirksamkeit wird an wachen, nüchternen männlichen Ratten (SPF-Wistar, Gewicht 180 bis 250 g) der Einfluss von erfindungsgemässen Verbindungen auf den

Phenolrot-Blutspiegel dargestellt. Nach der Methode von Kreppel, E. (Med. exp. 1 (1959), 285–289) erhalten je 8 Tiere 75 mg/kg Phenolrot in 5 ml/kg Kochsalzlösung intraperitoneal, nachdem ihnen 30 Minuten vorher entweder 10 mg/kg Tylose-Suspension (0,5%) als Kontrollen oder 100 mg/kg Prüfsubstanz in Tylose-Suspension verabreicht worden war. Plasma wird durch Punktion des retroorbitalen Venenplexus 30, 60 und 120 Minuten nach Gabe von Phenolrot, bzw. 60, 90 und 150 Minuten nach Substanzgabe gewonnen, mit NaOH versetzt und die Extinktion bei 546 nm im Photometer (Eppendorf) bestimmt.

Eine potentielle uricosurische Wirksamkeit liegt vor, wenn die Extinktionswerte signifikant höher als in der Kontrollgruppe sind.

Die neuen erfindungsgemässen Verbindungen sind als Arzneimittel verwendbare Substanzen. Sie bewirken bei oraler oder parenteraler Anwendung eine Steigerung der Wasser- und Salzausscheidung und können daher zur Behandlung oedematöser und hypertoner Zustände und zur Ausschwemmung toxischer Substanzen dienen. Darüber hinaus können die Verbindungen bei akutem Nierenversagen eingesetzt werden. Insbesondere zeigen sie auch eine vorteilhafte uricosurische Wirkung.

Die neuen Verbindungen können in bekannter Weise in die üblichen Formulierungen übergeführt werden, wie Tabletten, Kapseln, Dragees, Pillen, Granulate, Sirupe, Emulsionen, Suspensionen und Lösungen, unter Verwendung inerter, nichttoxischer, pharmazeutisch geeigneter Trägersubstanzen oder Lösungsmittel. Hierbei soll die therapeutisch wirksame Verbindung jeweils in einer Konzentration von etwa 0,5 bis 90 Gew.-% der Gesamtmischung vorhanden sein, d. h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z. B. im Fall der Benutzung von Wasser als Verdünnungsmittel gegebenenfalls organische Lösungsmittel als Hilfslösungsmittel verwendet werden können.

Als Hilfsstoffe seien beispielhaft aufgeführt:

Wasser, nichttoxische organische Lösungsmittel wie Paraffine (z. B. Erdölfraktionen), pflanzliche Öle (z. B. Erdnuss-/Sesamöl), Alkohole (z. B. Ethylalkohol, Glycerin), Glykole (z. B. Propylenglykol, Polyethylenglykol), feste Trägerstoffe wie z. B. natürliche Gesteinsmehle (z. B. Kaoline, Tonerden, Talkum, Kreide), synthetische Gesteinsmehle (z. B. hochdisperse Kieselsäure, Silikate), Zucker (z. B. Rohr-, Milch- und Traubenzukker), Emulgiermittel wie nichtionogene und anionische Emulgatoren (z. B. Polyethylen-Fettsäure-Ester, Polyoxyethylen-Fettalkohol-Ether, Alkylsulfonate und Arylsulfonate), Dispergiermittel (z. B. Lignin, Methylcellulose, Stärke und Polyvinylpyrrolidon) und Gleitmittel (z. B. Magnesiumstearat, Talkum, Stearinsäure und Natriumlaurylsulfat).

Die Applikation erfolgt in üblicher Weise, vorzugsweise oral oder parenteral.

Im Falle der oralen Anwendung können Tabletten selbstverständlich ausser den genannten Trägerstoffen auch Zusätze wie Natriumcitrat, Calciumcarbonat und Calciumphosphat zusammen mit verschiedenen Zuschlagstoffen wie Stärke, vorzugsweise Kartoffelstärke, Gelatine und dergleichen enthalten. Weiterhin können Gleitmittel wie Magnesiumstearat, Natriumlaurylsulfat und Talkum zum Tablettieren mitverwendet werden. Im Falle wässriger Suspensionen und/oder Elixieren, die für orale Anwendungen gedacht sind, können die Wirkstoffe ausser mit den o.g. Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden. Als besonders vorteilhaft für den Fall der parenteralen Anwendung hat sich die Tatsache herausgestellt, dass die erfindungsgemässen Verbindungen in Wasser gut lösliche Salze zu bilden vermögen. Diese Salze werden erhalten, wenn man die erfindungsgemässen Verbindungen in einem geeigneten Lösungsmittel mit der äquimolaren Menge einer nichttoxischen anorganischen oder organischen Base vereinigt. Als Beispiele seien genannt: Natronlauge, Kalilauge, Ethanolamin, Diethanolamin, Triethanolamin, Amino-tris-hydroxymethyl-methan, Glucosamin, N-Methylglucosamin. Derartige Salze können auch für die orale Anwendung der erfindungsgemässen Verbindungen eine erhöhte Bedeutung besitzen, indem sie die Resorption je nach Wunsch beschleunigen oder verzögern. Als Beispiele seien ausser den oben bereits erwähnten Salzen genannt: Magnesiumsalze, Calciumsalze, Aluminiumsalze und Eisensalze.

Im allgemeinen hat es sich als vorteilhaft erwiesen, bei parenteraler Applikation Mengen von etwa 0,05 bis 100 mg/kg, vorzugsweise etwa 0,1 bis 10 mg/kg Körpergewicht pro Tag zur Erzielung wirksamer Ergebnisse zu verabreichen, und bei oraler Applikation beträgt die Dosierung etwa 0,1 bis 500 mg/kg, vorzugsweise 0,5 bis 100 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Intervall, zu welchem die Verabreichung erfolgt. So kann es in wenigen Fällen ausreichend sein, mit weniger als der vorgenannten Mindestmenge auszukommen, während in anderen Fällen die genannte obere Grenze überschritten werden muss. Im Fall der Applikation grösserer Mengen kann es empfehlenswert sein, diese in mehrere Einzelgaben über den Tag zu verteilen.

Diese Angaben gelten sowohl für die Anwendung der erfindungsgemässen Verbindungen in der Veterinär- als auch in der Humanmedizin.

Die bei der Durchführung des erfindungsgemässen Herstellungsverfahrens eingesetzten Phosphonatverbindungen der allgemeinen Formel (III) sind bekannt oder können nach bekannten Methoden hergestellt werden (vgl. I. Shahak et al. Isr. J. Chem. 7, 585 (1969)).

Als starke Basen zur Verwendung bei der Durchführung der Verfahrensvariante a) seien beispielhaft genannt: Alkalihydride, wie Natriumhydrid, Kaliumhydrid, Lithiumhydrid und Alkalialkoholate wie Natriumethylat, Kaliumethylat oder Kaliummethylat oder Alkalimetallalkyle wie Methyllithium oder Butyllithium.

Die bei der Durchführung der Verfahrensvariante b) eingesetzten Acetamidderivate der allgemeinen Formel (IV) sind bekannt oder können nach bekannten Methoden hergestellt werden (vgl. a) Brit. Pat. 715 896 (1954); C.A. 49, 13290d (1955); b) DBP 1 142 859 (1960); C.A. 59, 7377c (1963)).

Bei dieser Verfahrensvariante b) werden vorzugsweise saure oder basische Katalysatoren eingesetzt, von denen beispielhaft genannt seien: basische Amine wie Dialkylamine, Piperidin oder Pyridin oder anorganische Säuren, insbesondere Salzsäure oder Kondensationsmittel wie Carbonsäureanhydride.

Die gemäss Verfahrensvariante c) eingesetzten Alkensäuren der allgemeinen Formel (V) sind bisher noch nicht bekannt geworden, lassen sich aber nach den oben angegebenen Verfahren in an sich bekannter Weise herstellen. Die für die Umsetzung mit Aminen der allgemeinen Formel (VI) zweckmässige Aktivierung der freien Carboxylgruppe erfolgt vorzugsweise über das entsprechende Säurehalogenid, insbesondere über das entsprechende Säurechlorid unter Verwendung von Halogenidbildnern, wie z. B. Thionylchlorid, Phosphortrichlorid und Phosphorpentachlorid.

Bei allen erfindungsgemässen Verfahren können als Verdünnungsmittel die üblichen inerten organischen Lösungsmittel eingesetzt werden. Hierzu gehören vorzugsweise Ether wie Diethylether, Glykolether wie Glykoldimethylether, Dioxan, Tetrahydrofuran oder Alkohole wie Methanol, Ethanol, Propanol, Butanol, Benzylalkohol oder Sulfoxide wie Dimethylsulfoxid, Basen wie Pyridin, Chinolin, Piccolin oder Kohlenwasserstoffe wie Benzol, Toluol, Xylol sowie Dimethylformamid.

Bei der Herstellung der Alkensäuren der allgemeinen Formel (V) werden für die Verseifung der korrespondierenden Ester als Basen vorzugsweise benutzt: Alkalimetallhydroxide wie Natriumhydroxid, Kaliumhydroxid, Erdalkalihydroxid wie Bariumhydroxid oder Calciumhydroxid.

Bei der Herstellung über die Aldehyde der allgemeinen Formel (VIII) mit Malonsäuren der allgemeinen Formel (IX) werden als Kondensationsmittel vorzugsweise verwendet: Pyridin, substituierte Pyridinderivate wie Dialkylaminopyridine, Chinolin, Isochinolin, Dialkylamine wie Dimethylamin, Dibutylamin, Pyrrolidin, Piperidin und ähnliche stickstoffhaltige organische Basen.

Die nachfolgenden Beispiele Nr. 1 bis 17 k erläutern die Herstellung der als Ausgangssubstanzen verwendeten Carbonylverbindungen der allgemeinen Formel (II) (Tabelle 1).

Die Beispiele 18 bis 33 m erläutern die Herstellung der erfindungsgemäss als Zwischenprodukte verwendeten Alkencarbonsäuren der allgemeinen Formel (V) (Tabelle 2).

Die dann folgenden Beispiele ab Nr. 34 erläutern die Herstellung der erfindungsgemässen Imidazoazolalkensäureamide der allgemeinen Formel (I) (Tabelle 3) als Endprodukte.

I. Beispiele für die Herstellung von Carbonylverbindungen der allgemeinen Formel (II) als Ausgangsprodukte.

Beispiel 1

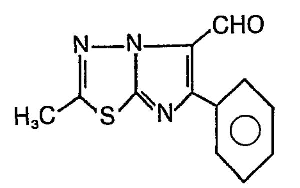

2-Methyl-6-phenyl-imidazo[2,1-b]-1,3,4-thiadiazol-5-carbaldehyd

Unter Kühlung werden zu 180 ml Dimethylformamid 60 ml Phosphoroxichlorid zugetropft. Dann gibt man weitere 400 ml Dimethylformamid und 130 g (0,6 Mol)

2-Methyl-6-phenyl-imidazo[2,1-b]-1,3,4-thiadiazol

zu und erwärmt 2 Stunden auf 100 °C. Der Ansatz wird auf 3 kg Eis gegeben, mit 410 g 40%iger Natronlauge versetzt und schnell auf 90 °C erhitzt. Nach 5 Minuten wird abgekühlt und abgesaugt. Ausbeute 132 g (90% der Theorie) vom Fp. 152 bis 153 °C.

In analoger Weise wurden die Beispiele der folgenden Tabelle 1 durch Umsetzung jeweils äquivalenter Mengen der Reaktionspartner hergestellt.

Tabelle 1

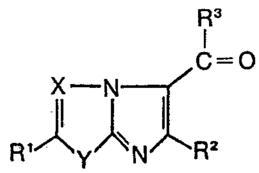

| Beispiel Nr. | X | Y | $R^1$ | $R^2$ | $R^3$ | Fp. °C | Ausbeute % d.Th. |
|---|---|---|---|---|---|---|---|
| 2 | CH | S | H | $C_6H_5$ | H | 128–9 | 25 |
| 3 | N | S | $CF_3$ | $C_6H_5$ | H | 186–8 | 32 |
| 4 | N | S | $C_2H_5$ | $C_6H_5$ | H | 101–2 | 28 |
| 5 | N | S | 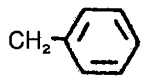 | $C_6H_5$ | H | 140–2 | 40 |
| 6 | N | S | $CH=CH-CH_3$ | $C_6H_5$ | H | 164–5 | 33 |
| 7 | N | S | 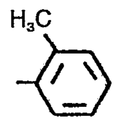 | $C_6H_5$ | H | 149 | 29 |
| 8 | N | S | H | $C_6H_5$ | H | 234–38 | 34 |
| 9 | N | O | $CH_3$ | $C_6H_5$ | H | 165 | 38 |
| 10 | N | NH | $CH_3$ | $C_6H_5$ | H | 270 | 36 |
| 11 | N | S | $CH_3$ | 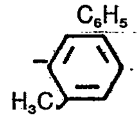 | H | 109–11 | 56 |
| 12 | N | S | $CH_3$ | 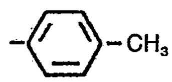 | H | 139–41 | 64 |
| 13 | N | S | $CH_3$ | 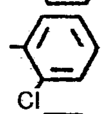 | H | 120–22 | 48 |
| 14 | N | S | $CH_3$ | 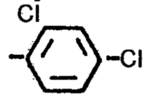 | H | 175–77 | 71 |
| 15 | N | S | $CH_3$ | 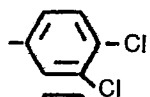 | H | 203–05 | 69 |
| 16 | N | S | $CH_3$ | 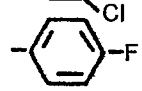 | H | 168–70 | 66 |
| 17 | N | S | $CH_3$ | 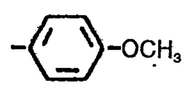 | H | 160–162 | 65 |
| 17a | N | S | $SO_2CH_3$ | $C_6H_5$ | H | 250 | 52 |
| 17b | N | S | $(CH_2)_2-O-C_2H_5$ | $C_6H_5$ | H | 57 | 40 |
| 17c | N | S | $(CH_2)_2-S-CH_3$ | $C_6H_5$ | H | 83 | 66 |
| 17d | N | S | $CO_2n-C_4H_9$ | $C_6H_5$ | H | 74 | 85 |
| 17e | N | S | 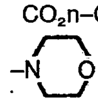 | $C_6H_5$ | H | 180–82 | 83 |
| 17f | N | S | 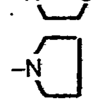 | $C_6H_5$ | H | 200–02 | 95 |
| 17g | N | S | 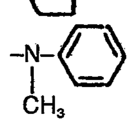 | $C_6H_5$ | H | 142–44 | 87 |
| 17h | N | S | 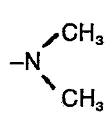 | $C_6H_5$ | H | 167–69 | 96 |

Tabelle 1 (Fortsetzung)

| Beispiel Nr. | X | Y | $R^1$ | $R^2$ | $R^3$ | Fp. °C | Ausbeute % d.Th. |
|---|---|---|---|---|---|---|---|
| 17i | N | S | 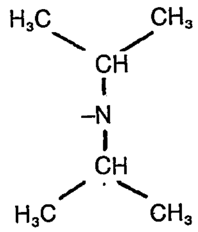 | $C_6H_5$ | H | 128–30 | 91 |
| 17j | N | S | $-CH_3$ | 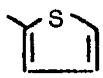 | H | 184–86 | 73 |
| 17k | N | S | $-CH_3$ | 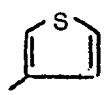 | H | 181–83 | 95 |

II. Beispiele für die Herstellung von Alkensäuren der allgemeinen Formel (V) als Zwischenprodukte.

Beispiel 18

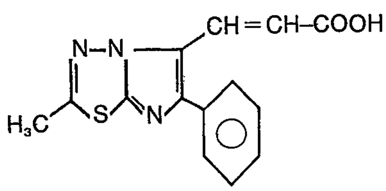

90 g 2-Methyl-6-phenyl-imidazo[2,1-b]-1,3,4-thiadiazol-5-carbaldehyd werden mit 60 g Malonsäure und 3 ml Piperidin in 95 ml Pyridin 10 Stunden unter Rückfluss gekocht. Man kühlt auf 5 °C ab, rührt 2 Stunden bei dieser Temperatur und saugt ab. Der Rückstand wird mit Wasser gewaschen und getrocknet. Man erhält 92 g (84% der Theorie) der

β-(2-Methyl-6-phenyl-imidazo[2,1-b]-1,3,4-thiadiazol-5-yl-propensäure

vom Schmelzpunkt 278–280 °C (Zersetzung).

In analoger Weise wurden die Beispiele der folgenden Tabelle durch Umsetzung jeweils äquivalenter Mengen der Reaktionspartner hergestellt.

Tabelle 2

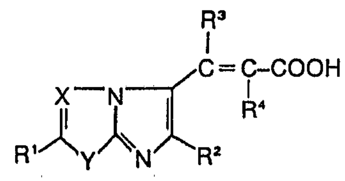

| Beispiel Nr. | X | Y | R¹ | R² | R³ | R⁴ | Lösungsmittel (für Umkrist.) | Fp. °C | Ausbeute % d. Th. |
|---|---|---|---|---|---|---|---|---|---|
| 19 | CH | S | H | $C_6H_5$ | H | H | DMF | 260–1 | 45 |
| 20 | N | S | $CF_3$ | $C_6H_5$ | H | H | Ethanol DMF | 340–2 | 77 |
| 21 | N | S | $C_2H_5$ | $C_6H_5$ | H | H | Ethanol DMF | 270–2 | 81 |
| 22 | N | S | $CH_2$- 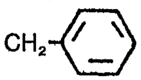 | $C_6H_5$ | H | H | Ethanol DMF | 274–6 | 66 |
| 23 | N | S | $CH=CH-CH_3$ | $C_6H_5$ | H | H | Ethanol DMF | 278–80 | 24 |
| 24 | N | S | 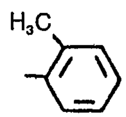 | $C_6H_5$ | H | H | Ethanol DMF | 297–300 | 53 |
| 25 | N | S | H | $C_6H_5$ | H | H | Ethanol DMF | 272–3 | 46 |
| 26 | N | S | $CH_3$ | $C_6H_5$ | H | $CH_3$ | Ethanol DMF | 298–300 | 79,5 |
| 27 | N | S | $CH_3$ | 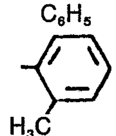 | H | H | Ethanol DMF | 251–53 | 49 |
| 28 | N | S | $CH_3$ | 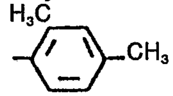 | H | H | Ethanol DMF | 260–62 | 58 |
| 29 | N | S | $CH_3$ | 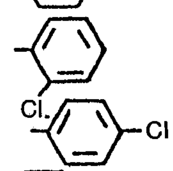 | H | H | Ethanol DMF | 235–37 | 63 |
| 30 | N | S | $CH_3$ |  | H | H | Ethanol DMF | 272–74 | 75 |
| 31 | N | S | $CH_3$ | 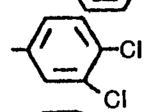 | H | H | Ethanol DMF | 255–57 | 81 |
| 32 | N | S | $CH_3$ | 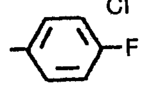 | H | H | Ethanol DMF | 273–75 | 58 |
| 33 | N | S | $CH_3$ | 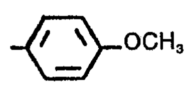 | H | H | Ethanol DMF | 279–81 | 62 |

Tabelle 2 (Fortsetzung)

| Beispiel Nr. | X | Y | $R^1$ | $R^2$ | $R^3$ | $R^4$ | Lösungsmittel (für Umkrist.) | Fp. °C | Ausbeute % d. Th. |
|---|---|---|---|---|---|---|---|---|---|
| 33a | N | S | 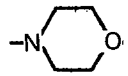 | $C_6H_5$ | H | H | Ethanol, DMF | 272–74 | 85 |
| 33b | N | S | 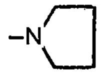 | $C_6H_5$ | H | H | Ethanol, DMF | > 280 | 73 |
| 33c | N | S | 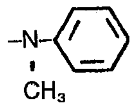 | $C_6H_5$ | H | H | Ethanol, DMF | 230–32 | 81 |
| 33d | N | S | 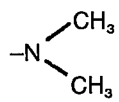 | $C_6H_5$ | H | H | Ethanol, DMF | 288–90 | 80 |
| 33e | N | S | $-CH_3$ | 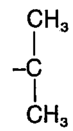 | H | H | Ethanol, DMF | 245–47 | 20 |
| 33f | N | S | 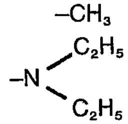 | $C_6H_5$ | H | H | Ethanol, DMF | 225–27 | 60 |
| 33g | N | S | 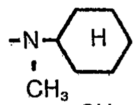 | $C_6H_5$ | H | H | Ethanol, DMF | 248–50 | 58 |
| 33h | N | S | 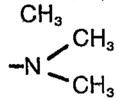 | $C_6H_5$ | H | H | Ethanol, DMF | 243–45 | 57 |
| 33i | N | S | 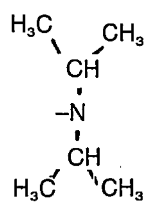 | $C_6H_5$ | H | H | Ethanol, DMF | 225–27 | 93 |
| 33j | N | S | $-CH_3$ | $-CH_3$ | H | H | Ethanol, DMF | 260–62 | 50 |
| 33k | N | S | $-CH_3$ | 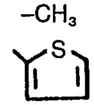 | H | H | Ethanol, DMF | > 280 | 78 |
| 33l | N | S | $-CH_3$ | 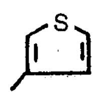 | H | H | Ethanol, DMF | > 280 | 73 |
| 33m | N | S | $-CH_3$ | $C_6H_5$ | H | H | Ethanol, DMF | 328 | 50 |

Die Verbindung des Tabellenbeispiels 26 wird erhalten, indem man 15 g

α-Methyl-β-(2-methyl-6-phenylimidazo-[2,1-b]-1,3,4-thiadiazol-5-yl-propensäureethylester

in 2,8 g Kaliumhydroxid, in 200 ml Ethanol und 20 ml Wasser 1 Stunde unter Rückfluss kocht. Man kühlt ab und engt im Vakuum ein. Der Rückstand wird in 300 ml Wasser gelöst und filtriert. Das Filtrat säuert man mit konzentrierter Salzsäure an, saugt den Niederschlag ab und kristallisiert aus Dimethylformamid um. Nach dem Trocknen erhält man

α-Methyl-β-(2-methyl-6-phenylimidazo[2,1-b]-1,3,4-thiadiazol-5-yl-propensäure

vom Schmelzpunkt 298 bis 300 °C (Ausbeute: 79,5% der Theorie).

III. Herstellung der erfindungsgemässen Endprodukte

Beispiel 34 (Verf. b)

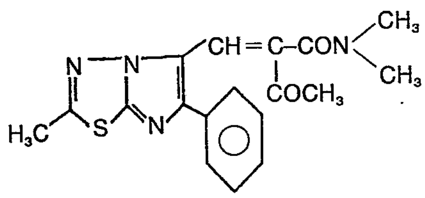

0,03 Mol 2-Methyl-6-phenylimidazo[2,1-b]-1,3,4-thiadiazol-5-carbaldehyd

werden in 0,1 Mol (13,0 g) Acetessigsäuredimethylamid und 0,6 ml Piperidin 4 Stunden auf 120 °C erhitzt. Anschliessend kühlt man ab und kristallisiert den entstandenen Niederschlag aus 30 ml Essigsäureethylester um. Nach dem Trocknen erhält man

α-Acetyl-β-(2-methyl-6-phenyl-imidazo[2,1-b]-1,3,4-thiadiazol-5-yl-propensäuredimethylamid

vom Schmelzpunkt 185 bis 187 °C.

Ausbeute: 43% der Theorie.

Beispiel 35

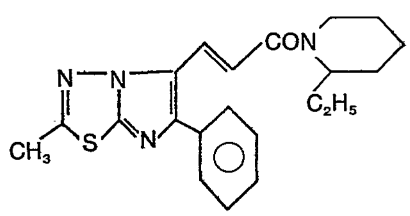

N-[β-(2-Methyl-6-phenyl-imidazo[2,1-b]-1,3,4-thiadiazol-5-yl-propenoyl]-2-ethyl-piperidin

143 g (0,5 Mol) β-(2-Methyl-6-phenyl-imidazo[2,1-b]-1,3,4-thiadiazol-5-yl-propensäure werden in 1,5 l abs. Toluol bei 60 bis 70 °C suspendiert. Man tropft 50 ml Thionylchlorid zu, wobei eine klare Lösung resultiert. Dann erhitzt man 2 Stunden unter Rückfluss und kühlt danach auf Raumtemperatur. Nun werden 220 ml (1,5 Mol) 2-Ethylpiperidin zugetropft und eine Stunde unter Rückfluss gekocht. Der gekühlte Ansatz wird abfiltriert (Rückstand wird verworfen), das Filtrat wird zweimal mit 300 ml Wasser extrahiert. Die Toluolphase wird über Natriumsulfat getrocknet, filtriert und destillativ eingeengt. Den Rückstand kristallisiert man aus 400 ml Essigsäureethylester um. Ausbeute 115 g (60% der Theorie) vom Fp. 143 bis 144 °C.

Beispiel 36

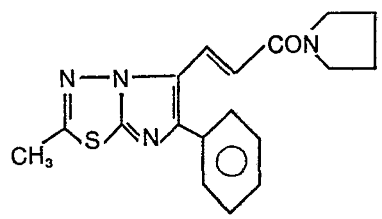

N-[β-(2-Methyl-6-phenylimidazo[2,1-b]-1,3,4-thiadiazol-5-yl)]propenoylpyrrolidin

Zu 30 ml abs. Benzol werden 0,6 g Natriumhydrid (80% in Öl) gegeben. Dann tropft man 5,5 g Diethylphosphonoessigsäurepyrrolidid zu, wobei die Temperatur bei 20 °C gehalten wird. Nachdem der Ansatz 1 Stunde bei Raumtemperatur gerührt wurde, gibt man weitere 40 ml ab. Benzol und portionsweise 4,8 g 2-Methyl-6-phenylimidazo[2,1-b]-1,3,4-thiadiazol-5-carbaldehyd zu und kocht 1 Stunde unter Rückfluss. Dann kühlt man ab und versetzt den Kristallbrei mit Wasser. Der Niederschlag wird abgesaugt und aus Propanol-2 umkristallisiert. Ausbeute 48% der Theorie; Fp. 210 bis 211 °C.

Tabelle 3

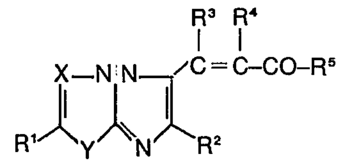

| Beispiel Nr. | X | Y | $R^1$ | $R^2$ | $R^3$ | $R^4$ | $R^5$ | Lösungsmittel (für Krist.) | Fp. °C | Ausbeute % d. Th. | analog Beispiel |
|---|---|---|---|---|---|---|---|---|---|---|---|
| 37 | N | S | $CH_3$ | $C_6H_5$ | H | H | 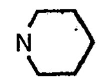 | Essigester | 188–91 | 62 | 35 |
| 38 | N | S | $CH_3$ | $C_6H_5$ | H | H | 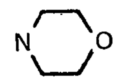 | Essigester | 217–19 | 54 | 36 |
| 39 | N | S | $CH_3$ | $C_6H_5$ | H | H | 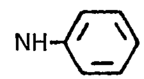 | Essigester | 242–43 | 46 | 35 |
| 40 | N | S | $CH_3$ | $C_6H_5$ | H | H | 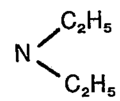 | Essigester | 154–5 | 57 | 35 |
| 41 | N | S | $CH_3$ | $C_6H_5$ | H | H | 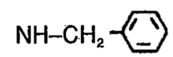 | Essigester | 194–6 | 49 | 35 |
| 42 | N | S | $CH_3$ | $C_6H_5$ | H | H | 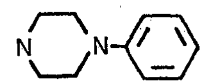 | Essigester | 196–7 | 54 | 35 |
| 43 | N | S | $CH_3$ | $C_6H_5$ | H | H | 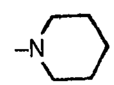 | Essigester | 148–50 | 49 | 35 |
| 44 | N | S | $CH_3$ | $C_6H_5$ | H | H | 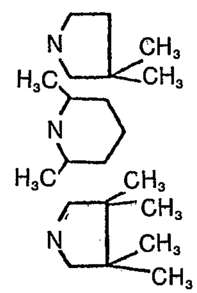 | Essigester | 206 | 58 | 35 |
| 45 | N | S | $CH_3$ | $C_6H_5$ | H | H |  | Essigester | 151 | 59 | 35 |
| 46 | N | S | $CH_3$ | $C_6H_5$ | H | H |  | Propanol-2 | 258–9 | 28 | 35 |
| 47 | N | S | $CH_3$ | $C_6H_5$ | H | H | 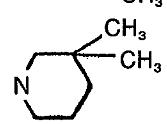 | Essigester | 138–40 | 61 | 35 |

Tabelle 3 (Fortsetzung)

| Beispiel Nr. | X | Y | $R^1$ | $R^2$ | $R^3$ | $R^4$ | $R^5$ | Lösungsmittel (für Krist.) | Fp. °C | Ausbeute % d.Th. | analog Beispiel |
|---|---|---|---|---|---|---|---|---|---|---|---|
| 48 | N | S | $CH_3$ | $C_6H_5$ | H | H | 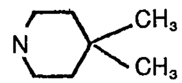 | Propanol-2 | 238–40 | 64 | 35 |
| 49 | N | S | $CH_3$ | $C_6H_5$ | H | H | 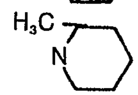 | Essigester | 170–1 | 46 | 36 |
| 50 | CH | S | H | $C_6H_5$ | H | H | 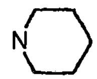 | Essigester | 175–6 | 49 | 35 |
| 51 | N | S | $CF_3$ | $C_6H_5$ | H | H | 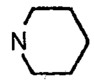 | Essigester | 202–4 | 68 | 35 |
| 52 | N | S | $CF_3$ | $C_6H_5$ | H | H | 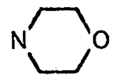 | Essigester | 248–51 | 62 | 35 |
| 53 | N | S | $CF_3$ | $C_6H_5$ | H | H | 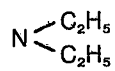 | Propanol-2 | 144–7 | 55 | 36 |
| 54 | N | S | $CH_3$ | $C_6H_5$ | H | H | 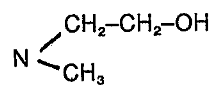 | Essigester | 187–8 | 47 | 35 |
| 55 | N | S | $CH_3$ | $C_6H_5$ | H | H | 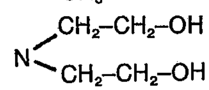 | Essigester | 179–80 | 49 | 35 |
| 56 | N | S | $CH_3$ | $C_6H_5$ | H | H | 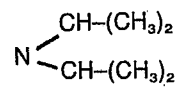 | Essigester | 194–6 | 58 | 35 |
| 57 | N | S | $CH_3$ | $C_6H_5$ | H | H | 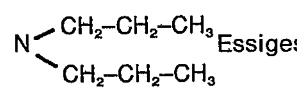 | Essigester | 168–71 | 61 | 35 |
| 58 | N | S | $CH_3$ | $C_6H_5$ | H | H | 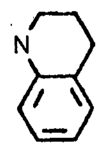 | Ethanol | 203–4 | 62 | 35 |
| 59 | N | S | $CH_3$ | $C_6H_5$ | H | H | 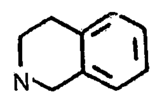 | Ethanol | 155–60 | 49 | 35 |

Tabelle 3 (Fortsetzung)

| Beispiel Nr. | X | Y | $R^1$ | $R^2$ | $R^3$ | $R^4$ | $R^5$ | Lösungsmittel (für Krist.) | Fp. °C | Ausbeute % d. Th. | analog Beispiel |
|---|---|---|---|---|---|---|---|---|---|---|---|
| 60 | N | S | $C_2H_5$ | $C_6H_5$ | H | H | 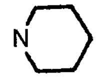 | Essigester | 181–2 | 54 | 36 |
| 61 | N | S | $C_2H_5$ | $C_6H_5$ | H | H | 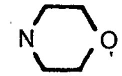 | Essigester | 180–2 | 54 | 36 |
| 62 | N | S | $C_2H_5$ | $C_6H_5$ | H | H | 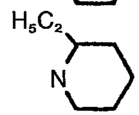 | Essigester | 100–4 | 62 | 35 |
| 63 | N | S | $CH_3$ | $C_6H_5$ | H | H | 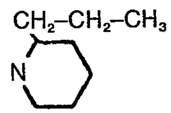 | Essigester | Öl | 24 | 35 |
| 64 | N | S | $CH_3$ | $C_6H_5$ | H | H | 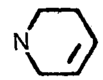 | Essigester | 218–9 | 52 | 35 |
| 65 | N | S | $CH_3$ | $C_6H_5$ | H | H | 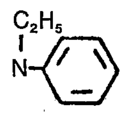 | Essigester | 237–9 | 65 | 35 |
| 66 | N | S | 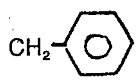 | $C_6H_5$ | H | H | 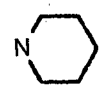 | Essigester | 155 | 24 | 36 |
| 67 | N | S | 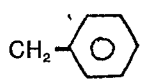 | $C_6H_5$ | H | H | 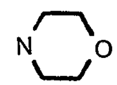 | Essigester | 160–3 | 42 | 36 |
| 68 | N | S | 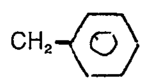 | $C_6H_5$ | H | H | 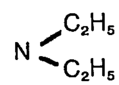 | Essigester | 124–5 | 48 | 36 |
| 69 | N | S | H | $C_6H_5$ | H | H | 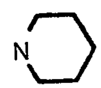 | Essigester | 183–5 | 61 | 36 |
| 70 | N | S | $CH=CH-CH_3$ | $C_6H_5$ | H | H | 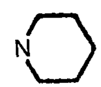 | Essigester | 216–8 | 54 | 36 |
| 71 | N | S | H | $C_6H_5$ | H | H | 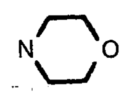 | Essigester | 222–3 | 61 | 36 |

Tabelle 3 (Fortsetzung)

| Beispiel Nr. | X | Y | $R^1$ | $R^2$ | $R^3$ | $R^4$ | $R^5$ | Lösungsmittel (für Krist.) | Fp. °C | Ausbeute % d. Th. | analog Beispiel |
|---|---|---|---|---|---|---|---|---|---|---|---|
| 72 | N | S | H | $C_6H_5$ | H | H | 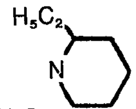 | Essigester | 160–1 | 42 | 35 |
| 75 | N | S | 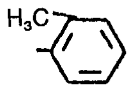 | $C_6H_5$ | H | H | 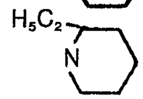 | Essigester | 84–8 | 55 | 35 |
| 76 | N | S | $CH=CH-CH_3$ | $C_6H_5$ | H | H | 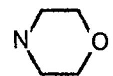 | Essigester | 188–90 | 42 | 36 |
| 77 | N | S | $CH_3$ | $C_6H_5$ | H | $CH_3$ | 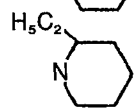 | Essigester | 109–10 | 29 | 35 |
| 78 | N | S | $CH_3$ | $C_6H_5$ | H | $CH_3$ | 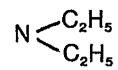 | Essigester | 129–30 | 49 | 35 |
| 79 | N | S | $CH_3$ | $C_6H_5$ | H | $CH_3$ | 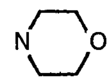 | Essigester | 135–8 | 49 | 35 |
| 80 | N | S | $CH_3$ | 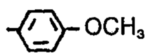 | H | H | 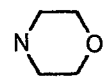 | Essigester | 190–92 | 52 | 35 |
| 81 | N | S | $CH_3$ | 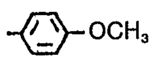 | H | H | 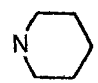 | Essigester | 210–12 | 42 | 35 |
| 82 | N | S | $CH_3$ | 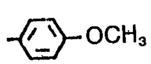 | H | H | 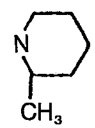 | Essigester | 135–37 | 53 | 35 |
| 83 | N | S | $CH_3$ | 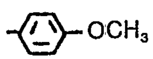 | H | H | 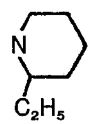 | Essigester | 83–85 | 49 | 35 |
| 84 | N | S | $CH_3$ | 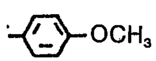 | H | H | 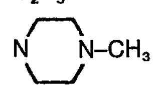 | Essigester | 166–68 | 52 | 35 |
| 85 | N | S | $CH_3$ | 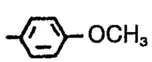 | H | H | 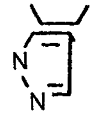 | Toluol | 223–25 | 67 | 35 |

Tabelle 3 (Fortsetzung)

| Beispiel Nr. | X | Y | $R^1$ | $R^2$ | $R^3$ | $R^4$ | $R^5$ | Lösungsmittel (für Krist.) | Fp. °C | Ausbeute % d. Th. | analog Beispiel |
|---|---|---|---|---|---|---|---|---|---|---|---|
| 86 | N | S | $CH_3$ | 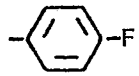 | H | H | 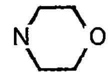 | Essigester | 184–86 | 25 | 36 |
| 87 | N | S | $CH_3$ | 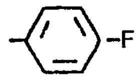 | H | H | 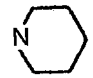 | Essigester | 173–75 | 48 | 36 |
| 88 | N | S | $CH_3$ | 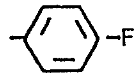 | H | H | 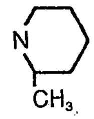 | Essigester | 155–57 | 51 | 35 |
| 89 | N | S | $CH_3$ | 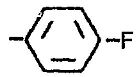 | H | H | 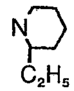 | Essigester | 135–37 | 63 | 35 |
| 90 | N | S | $CH_3$ | 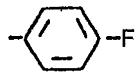 | H | H | 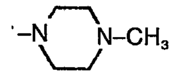 | Essigester | 171–73 | 45 | 35 |
| 91 | N | S | $CH_3$ | 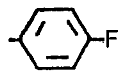 | H | H | 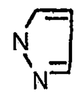 | Essigester | 195–97 | 58 | 35 |
| 92 | N | S | $CH_3$ | 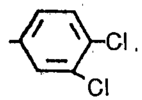 | H | H | 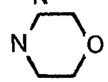 | Essigester | 197–99 | 62 | 35 |
| 93 | N | S | $CH_3$ | 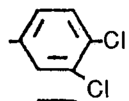 | H | H | 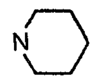 | Essigester | 155–57 | 51 | 36 |
| 94 | N | S | $CH_3$ | 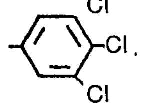 | H | H | 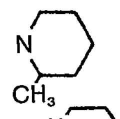 | Essigester | 158–60 | 46 | 35 |
| 95 | N | S | $CH_3$ | 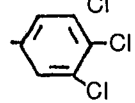 | H | H | 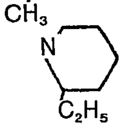 | Essigester | 117–19 | 49 | 35 |
| 96 | N | S | $CH_3$ | 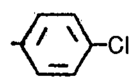 | H | H | 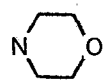 | Essigester | 176–78 | 49 | 35 |
| 97 | N | S | $CH_3$ | 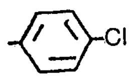 | H | H | 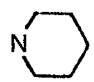 | Essigester | 180–82 | 57 | 36 |

Tabelle 3 (Fortsetzung)

| Beispiel Nr. | X | Y | $R^1$ | $R^2$ | $R^3$ | $R^4$ | $R^5$ | Lösungsmittel (für Krist.) | Fp. °C | Ausbeute % d. Th. | analog Beispiel |
|---|---|---|---|---|---|---|---|---|---|---|---|
| 98 | N | S | $CH_3$ | 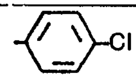 | H | H | 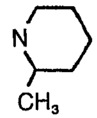 | Essigester | 140–42 | 54 | 35 |
| 99 | N | S | $CH_3$ | 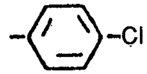 | H | H | 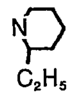 | Essigester | 125–27 | 48 | 35 |
| 100 | N | S | $CH_3$ | 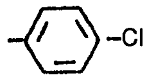 | H | H | 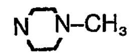 | Essigester | 128–30 | 55 | 35 |
| 101 | N | S | $CH_3$ | 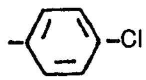 | H | H | 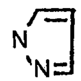 | Essigester | 238–40 | 29 | 35 |
| 102 | N | S | $CH_3$ | 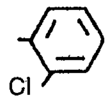 | H | H | 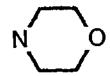 | Essigester | 210–12 | 25 | 36 |
| 103 | N | S | $CH_3$ | 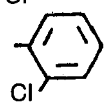 | H | H | 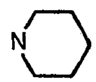 | Essigester | 160–62 | 29 | 36 |
| 104 | N | S | $CH_3$ | 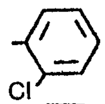 | H | H | 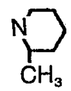 | Essigester | 138–40 | 31 | 35 |
| 105 | N | S | $CH_3$ | 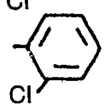 | H | H | 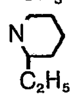 | Essigester | 145–47 | 48 | 35 |
| 106 | N | S | $CH_3$ | 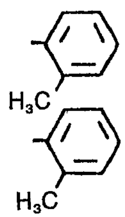 | H | H | 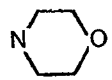 | Essigester | 189–91 | 49 | 36 |
| 107 | N | S | $CH_3$ |  | H | H | 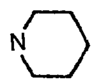 | Essigester | 178–80 | 44 | 36 |
| 108 | N | S | $CH_3$ | 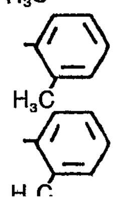 | H | H | 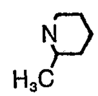 | Essigester | 136–38 | 54 | 35 |
| 109 | N | S | $CH_3$ |  | H | H | 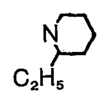 | Essigester | 77–80 | 59 | 35 |

Tabelle 3 (Fortsetzung)

| Beispiel Nr. | X | Y | $R^1$ | $R^2$ | $R^3$ | $R^4$ | $R^5$ | Lösungsmittel (für Krist.) | Fp. °C | Ausbeute % d.Th. | analog Beispiel |
|---|---|---|---|---|---|---|---|---|---|---|---|
| 110 | N | S | $CH_3$ | 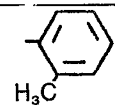 | H | H | 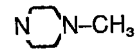 | Essigester | 143–44 | 53 | 35 |
| 111 | N | S | $CH_3$ | 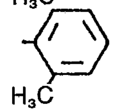 | H | H | 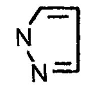 | Essigester | 180–82 | 61 | 35 |
| 112 | N | S | $CH_3$ | 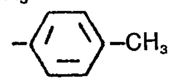 | H | H | 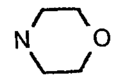 | Essigester | 160–62 | 64 | 36 |
| 113 | N | S | $CH_3$ | 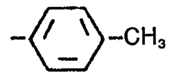 | H | H | 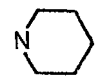 | Essigester | 178–80 | 50 | 36 |
| 114 | N | S | $CH_3$ | 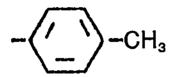 | H | H | 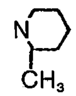 | Essigester | 118–21 | 61 | 35 |
| 115 | N | S | $CH_3$ | 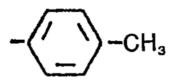 | H | H | 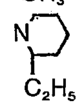 | Essigester | 125–27 | 42 | 35 |
| 116 | N | S | $CH_3$ | $C_6H_5$ | H | H | $NHC_4H_9$ | Essigester | 126 | 52 | 35 |
| 117 | N | S | $CH_3$ | $C_6H_5$ | H | H | 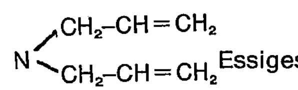 | Essigester | 135 | 60 | 35 |
| 118 | N | S | $CH_3$ | $C_6H_5$ | H | H | 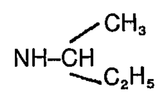 | Essigester | 200 | 71 | 35 |
| 119 | N | S | $CH_3$ | $C_6H_5$ | H | H | 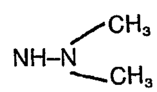 | Essigester | 201 | 47 | 35 |
| 120 | N | S | $CH_3$ | $C_6H_5$ | H | H | $NH_2$ | Essigester | 260–1 | 47 | 35 |
| 121 | N | S | $CH_3$ | $C_6H_5$ | H | CN | $NH_2$ | Essigester | 195–8 | 80 | 34 |
| 122 | N | S | $CH_3$ | $C_6H_5$ | H | CN | 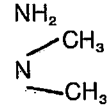 | Essigester | 199–201 | 65 | 34 |

Tabelle 3 (Fortsetzung)

| Beispiel Nr. | X | Y | $R^1$ | $R^2$ | $R^3$ | $R^4$ | $R^5$ | Lösungsmittel (für Krist.) | Fp. °C | Ausbeute % d.Th. | analog Beispiel |
|---|---|---|---|---|---|---|---|---|---|---|---|
| 123 | N | S | $CH_3$ | $C_6H_5$ | H | H | 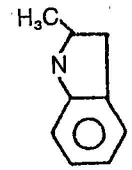 | Essigester | 185–6 | 32 | 35 |
| 124 | N | S | $CH_3$ | $C_6H_5$ | H | CN | 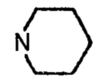 | Essigester | 237–8 | 75 | 34 |
| 125 | N | S | $CH_3$ | $C_6H_5$ | H | H | 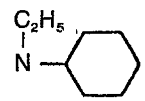 | Essigester | 170–1 | 45 | 35 |
| 126 | N | S | $CH_3$ | $C_6H_5$ | H | H | 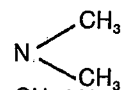 | Essigester | 215 | 54 | 35 |
| 127 | N | S | $CH_3$ | $C_6H_5$ | H | H | 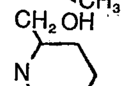 | Essigester | 210 | 39 | 35 |
| 128 | N | S | $CH_3$ | $C_6H_5$ | H | H | 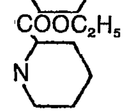 | Essigester | 166 | 28 | 35 |
| 129 | N | S | $CH_3$ | $C_6H_5$ | H | H | 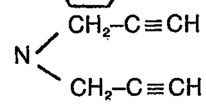 | Essigester | 277 | 55 | 35 |
| 130 | N | S | $CH_3$ | $C_6H_5$ | H | H | 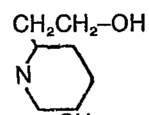 | Essigester | 219 | 43 | 35 |
| 131 | N | S | $CH_3$ | $C_6H_5$ | H | H | 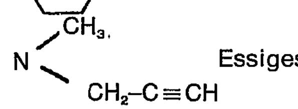 | Essigester | 230–31 | 61 | 35 |
| 132 | N | S | $CH_3$ | $C_6H_5$ | H | H | 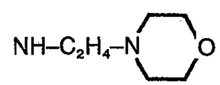 | Essigester | 110 | 29 | 35 |
| 133 | N | O | $CH_3$ | $C_6H_5$ | H | H | 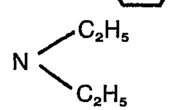 | Essigester | 168–70 | 41 | 36 |

Tabelle 3 (Fortsetzung)

| Beispiel Nr. | X | Y | $R^1$ | $R^2$ | $R^3$ | $R^4$ | $R^5$ | Lösungsmittel (für Krist.) | Fp. °C | Ausbeute % d.Th. | analog Beispiel |
|---|---|---|---|---|---|---|---|---|---|---|---|
| 134 | N | NH | $CH_3$ | $C_6H_5$ | H | H | 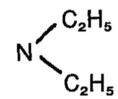 | Dimethylformamid | 270–72 | 18 | 36 |

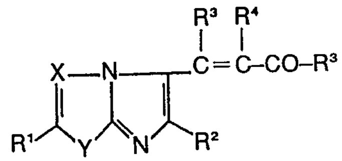

| | | | | | | | | | | | |
|---|---|---|---|---|---|---|---|---|---|---|---|
| 135 | N | S | 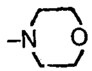 | $C_6H_5$ | H | H | 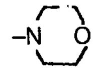 | Isopropanol | 203–05 | 66 | 35 |
| 136 | N | S | 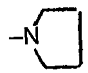 | $C_6H_5$ | H | H | 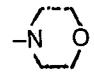 | isopropanol | 288–90 | 83 | 35 |
| 137 | N | S | 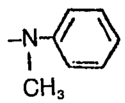 | $C_6H_5$ | H | H | 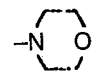 | Isopropanol | 163–65 | 61 | 35 |
| 138 | N | S | $-CH_3$ | $-CH_3$ | H | H | 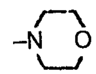 | Isopropanol | 130–32 | 19 | 35 |
| 139 | N | S | 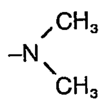 | $C_6H_5$ | H | H | 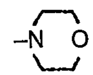 | Isopropanol | 233–35 | 79 | 35 |
| 140 | N | S | 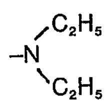 | $C_6H_5$ | H | H | 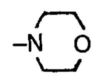 | Isopropanol | 133–35 | 70 | 35 |
| 141 | N | S | 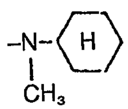 | $C_6H_5$ | H | H | 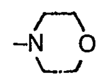 | Isopropanol | 125–27 | 71 | 35 |
| 142 | N | S | 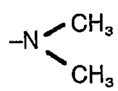 | $C_6H_5$ | H | H | 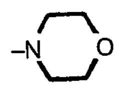 | Isopropanol | 133–35 | 80 | 35 |

Tabelle 3 (Fortsetzung)

| Beispiel Nr. | X | Y | $R^1$ | $R^2$ | $R^3$ | $R^4$ | $R^5$ | Lösungsmittel (für Krist.) | Fp. °C | Ausbeute % d.Th. | analog Beispiel |
|---|---|---|---|---|---|---|---|---|---|---|---|
| 143 | N | S | 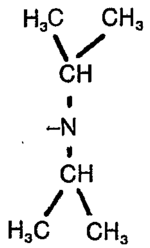 | $C_6H_5$ | H | H | 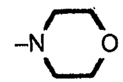 | Isopropanol | 188–90 | 54 | 35 |
| 144 | N | S | $-CH_3$ | 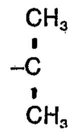 | H | H | 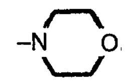 | Isopropanol | 240–42 | 58 | 35 |
| 145 | N | S | $-CH_3$ | 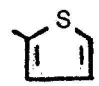 | H | H | 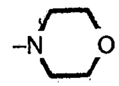 | Isopropanol | 185–87 | 70 | 35 |
| 146 | N | S | $-CH_3$ | $-CH_3$ | H | H | 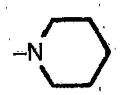 | Isopropanol | 100–02 | 26 | 35 |
| 147 | N | S | $-CH_3$ | 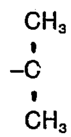 | H | H | 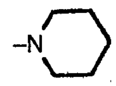 | Isopropanol | 135–37 | 19 | 35 |
| 148 | N | S | $-CH_3$ | 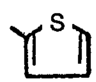 | H | H | 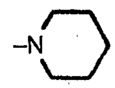 | Isopropanol | 185–87 | 48 | 35 |
| 149 | N | S | 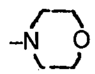 | $C_6H_5$ | H | H | 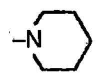 | Isopropanol | 205–07 | 22 | 35 |
| 150 | N | S | 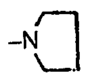 | $C_6H_5$ | H | H | 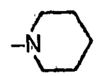 | Isopropanol | 275–77 | 75 | 35 |
| 151 | N | S | 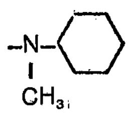 | $C_6H_5$ | H | H | 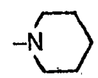 | Isopropanol | 143–45 | 54 | 35 |
| 152 | N | S | 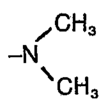 | $C_6H_5$ | H | H | 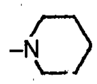 | Isopropanol | 198–200 | 85 | 35 |

Tabelle 3 (Fortsetzung)

| Beispiel Nr. | X | Y | R¹ | R² | R³ | R⁴ | R⁵ | Lösungsmittel (für Krist.) | Fp. °C | Ausbeute % d.Th. | analog Beispiel |
|---|---|---|---|---|---|---|---|---|---|---|---|
| 153 | N | S | 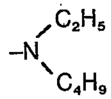 | $C_6H_5$ | H | H | 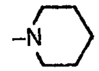 | Isopropanol | 113–15 | 63 | 35 |
| 154 | N | S | 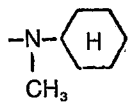 | $C_6H_5$ | H | H | 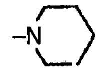 | Isopropanol | 170–72 | 23 | 35 |
| 155 | N | S | 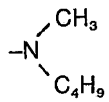 | $C_6H_5$ | H | H | 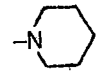 | Isopropanol | 123–25 | 88 | 35 |
| 156 | N | S | 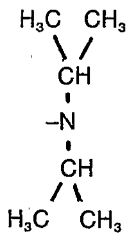 | $C_6H_5$ | H | H | 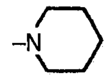 | Isopropanol | 170–72 | 84 | 35 |
| 157 | N | S | $-CH_3$ | 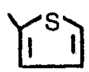 | H | H | 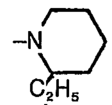 | Isopropanol | 140–42 | 74 | 35 |
| 158 | N | S | 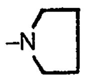 | $C_6H_5$ | H | H | 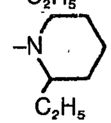 | Isopropanol | 165–67 | 76 | 35 |
| 159 | N | S | 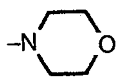 | $C_6H_5$ | H | H | 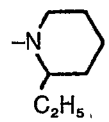 | Isopropanol | 175–77 | 56 | 35 |
| 160 | N | S | 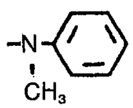 | $C_6H_5$ | H | H | 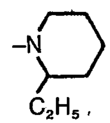 | Isopropanol | 155–57 | 85 | 35 |
| 161 | N | S | 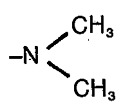 | $C_6H_5$ | H | H | 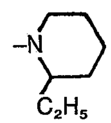 | Isopropanol | 143–45 | 86 | 35 |

Tabelle 3 (Fortsetzung)

| Beispiel Nr. | X | Y | R¹ | R² | R³ | R⁴ | R⁵ | Lösungsmittel (für Krist.) | Fp. °C | Ausbeute % d.Th. | analog Beispiel |
|---|---|---|---|---|---|---|---|---|---|---|---|
| 162 | N | S | 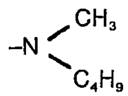 | $C_6H_5$ | H | H | 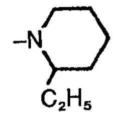 | Isopropanol | 118–20 | 56 | 35 |
| 163 | N | S | $-CH_3$ | 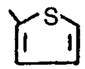 | H | H | 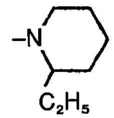 | Isopropanol | 130–32 | 67 | 35 |
| 164 | N | S | $-CH_3$ | 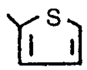 | H | H | 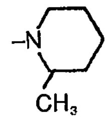 | Isopropanol | 166–68 | 75 | 35 |
| 165 | N | S | $-CH_3$ | 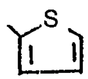 | H | H | 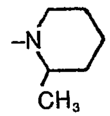 | Isopropanol | 168–70 | 26 | 35 |
| 166 | N | S | 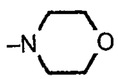 | $C_6H_5$ | H | H | 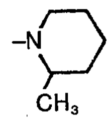 | Isopropanol | 168–70 | 35 | 35 |
| 167 | N | S | 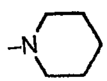 | $C_6H_5$ | H | H | 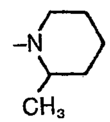 | Isopropanol | 210–12 | 16 | 35 |
| 168 | N | S | 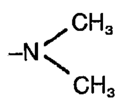 | $C_6H_5$ | H | H | 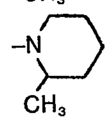 | Isopropanol | 160–62 | 37 | 35 |
| 169 | N | S | 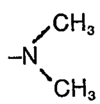 | $C_6H_5$ | H | H | 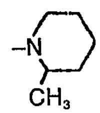 | Isopropanol | 138–40 | 95 | 35 |
| 170 | N | S | 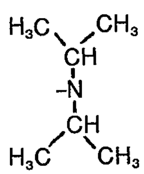 | $C_6H_5$ | H | H | 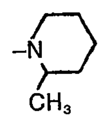 | Isopropanol | 148–50 | 81 | 35 |

Tabelle 3 (Fortsetzung)

| Beispiel Nr. | X | Y | $R^1$ | $R^2$ | $R^3$ | $R^4$ | $R^5$ | Lösungsmittel (für Krist.) | Fp. °C | Ausbeute % d. Th. | analog Beispiel |
|---|---|---|---|---|---|---|---|---|---|---|---|
| 171 | N | S | 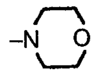 | $C_6H_5$ | H | H | 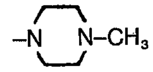 | Isopropanol | 203–05 | 34 | 35 |
| 172 | N | S | 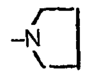 | $C_6H_5$ | H | H | 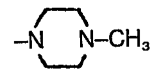 | Isopropanol | 213–15 | 60 | 35 |
| 173 | N | S | 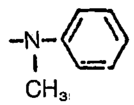 | $C_6H_5$ | H | H | 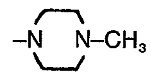 | Isopropanol | 165–67 | 84 | 35 |
| 174 | N | S | 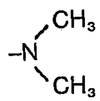 | $C_6H_5$ | H | H | 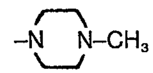 | Isopropanol | 205–07 | 44 | 35 |
| 175 | N | S | 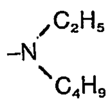 | $C_6H_5$ | H | H | 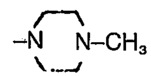 | Isopropanol | 127–29 | 77 | 35 |
| 176 | N | S | 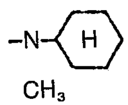 | $C_6H_5$ | H | H | 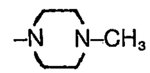 | Isopropanol | 148–50 | 83 | 35 |
| 177 | N | S | 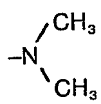 | $C_6H_5$ | H | H | 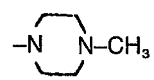 | Isopropanol | 125–27 | 93 | 35 |
| 178 | N | S | 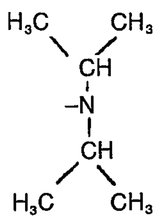 | $C_6H_5$ | H | H | 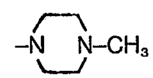 | Isopropanol | 187–89 | 77 | 35 |
| 179 | N | S | $-CH_3$ | 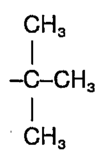 | H | H | 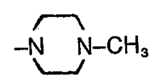 | Isopropanol | 147–49 | 69 | 35 |

Tabelle 3 (Fortsetzung)

| Beispiel Nr. | X | Y | R¹ | R² | R³ | R⁴ | R⁵ | Lösungsmittel (für Krist.) | Fp. °C | Ausbeute % d. Th. | analog Beispiel |
|---|---|---|---|---|---|---|---|---|---|---|---|
| 180 | N | S | $-CH_3$ | 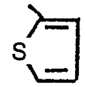 | H | H | 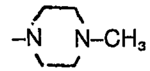 | Isopropanol | 160–62 | 60 | 35 |
| 181 | N | S | $-CH_3$ | 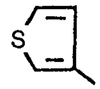 | H | H | 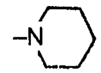 | Isopropanol | 203–05 | 87 | 35 |
| 182 | N | S | 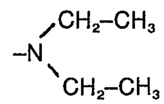 | $C_6H_5$ | H | H | 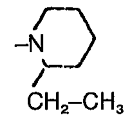 | Isopropanol | 146 | 45 | 35 |
| 183 | N | S | 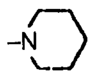 | $C_6H_5$ | H | H | 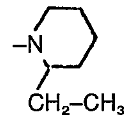 | Isopropanol | 185 | 30 | 35 |
| 184 | N | S | $-CO_2nC_4H_9$ | $C_6H_5$ | H | H | 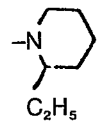 | Essigester | 120 | 50 | 36 |
| 185 | N | S | $-COOH$ | $C_6H_5$ | H | H | 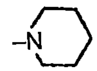 | Essigester | 160 | 80 | 36 |
| 186 | N | S | $-COOH$ | $C_6H_5$ | H | H | 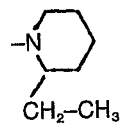 | Essigester | 148 | 80 | 36 |
| 187 | N | S | $-CH_2OH$ | $C_6H_5$ | H | H | 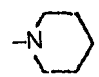 | Essigester | 194 | 48 | 36 |
| 188 | N | S | $-CH_2OH$ | $C_6H_5$ | H | H | 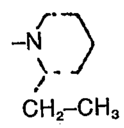 | Essigester | 163 | 50 | 36 |
| 189 | N | S | 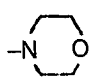 | $C_6H_5$ | H | H | 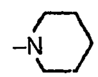 | Essigester | 230 | 60 | 36 |
| 190 | N | S | 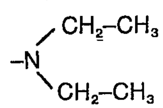 | $C_6H_5$ | H | H | 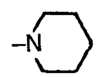 | Essigester | 170 | 65 | 36 |

Tabelle 3 (Fortsetzung)

| Beispiel Nr. | X | Y | R¹ | R² | R³ | R⁴ | R⁵ | Lösungsmittel (für Krist.) | Fp. °C | Ausbeute % d.Th. | analog Beispiel |
|---|---|---|---|---|---|---|---|---|---|---|---|
| 191 | N | S | 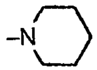 | $C_6H_5$ | H | H | 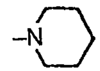 | Essigester | 220 | 60 | 36 |
| 192 | N | S | $-CH_3$ | $C_6H_5$ | H | H | 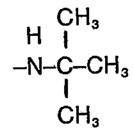 | Toluol | 253 | 31 | 35 |
| 193 | N | S | $-CH_3$ | $C_6H_5$ | H | $OC_2H_5$ | 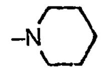 | Isopropanol | 178 | 25 | 36 |
| 194 | N | S | $-CO_2CH_3$ | $C_6H_5$ | H | H | 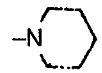 | Essigester | 160 | 55 | 36 |
| 195 | N | S | $-CO_2CH_3$ | $C_6H_5$ | H | H | 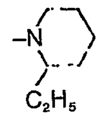 | Essigester | 142 | 50 | 36 |
| 196 | N | S | $-CO_2nC_4H_9$ | $C_6H_5$ | H | H | 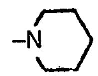 | Essigester | 114 | 60 | 36 |
| 197 | N | S | $-CO_2C_2H_5$ | $C_6H_5$ | H | H | 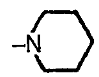 | Essigester | 146 | 80 | 36 |
| 198 | N | S | $-CO_2CH(CH_3)_2$ | $C_6H_5$ | H | H | 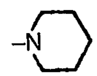 | Essigester | 135 | 90 | 36 |
| 199 | N | S | $-CH_3$ | $C_6H_5$ | H | H | 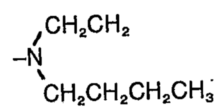 | Cyclohexan | 140 | 55 | 35 |
| 200 | N | S | $-CH_3$ | $C_6H_5$ | H | H | 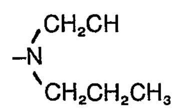 | Toluol | 152 | 65 | 35 |
| 201 | N | S | $-CH_3$ | $C_6H_5$ | H | H | 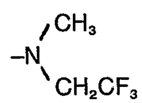 | Toluol | 180 | 45 | 35 |
| 202 | N | S | $-CH_3$ | $C_6H_5$ | H | H | 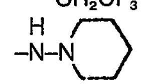 | Toluol | 230 | 50 | 35 |

Tabelle 3 (Fortsetzung)

| Beispiel Nr. | X | Y | $R^1$ | $R^2$ | $R^3$ | $R^4$ | $R^5$ | Lösungsmittel (für Krist.) | Fp. °C | Ausbeute % d. Th. | analog Beispiel |
|---|---|---|---|---|---|---|---|---|---|---|---|
| 203 | N | S | $-CH_3$ | $C_5H_5$ | H | H | 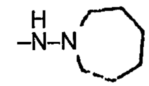 | Toluol | 200 | 70 | 35 |
| 204 | N | S | $-CH_3$ | $C_6H_5$ | H | H | $-\overset{H}{N}-CH_2CH_2CH_3$ | Toluol | 190 | 30 | 35 |
| 205 | N | S | $-CH_3$ | $C_6H_5$ | H | H | 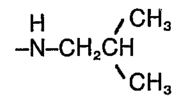 | Toluol | 165 | 33 | 35 |
| 206 | N | S | $-(CH_2)_2SCH_3$ | $C_6H_5$ | H | H | 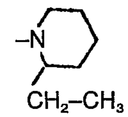 | Isopropanol | 96 | 15 | 36 |
| 207 | N | S | $-CH_3$ | $C_6H_5$ | H | H | 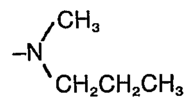 | Toluol | 156 | 51 | 35 |
| 208 | N | S | $-CH_3$ | $C_6H_5$ | H | H | 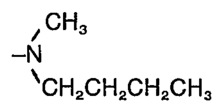 | Toluol | 128 | 60 | 35 |
| 209 | N | S | $-CH_3$ | $C_6H_5$ | H | H | 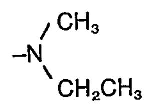 | Toluol | 190 | 55 | 35 |
| 210 | N | S | $-CH_3$ | $C_6H_5$ | H | F | 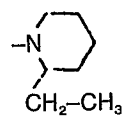 | Isopropanol | 130 | 34 | 35 |
| 211 | N | S | $-CH_3$ | $C_6H_5$ | H | H | 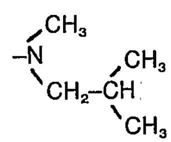 | Toluol | 131 | 50 | 35 |
| 212 | N | S | $-CH_3$ | $C_6H_5$ | H | H | 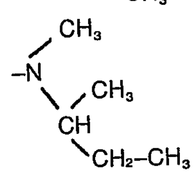 | Toluol | 131 | 55 | 35 |

Tabelle 3 (Fortsetzung)

| Beispiel Nr. | X | Y | $R^1$ | $R^2$ | $R^3$ | $R^4$ | $R^5$ | Lösungsmittel (für Krist.) | Fp. °C | Ausbeute % d.Th. | analog Beispiel |
|---|---|---|---|---|---|---|---|---|---|---|---|
| 213 | N | S | $-CH_3$ | $C_6H_5$ | H | H | 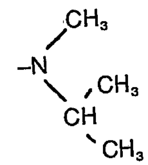 | Toluol | 160 | 40 | 35 |
| 214 | N | S | $-CH_3$ | $C_6H_5$ | H | H | 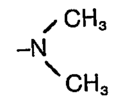 | Isopropanol | 215 | 80 | 35 |
| 215 | N | S | $-CH_3$ | $C_6H_5$ | H | H | $NH-CH_2CH_2CCH_3$ | Isopropanol | 95 | 67 | 35 |
| 216 | N | S | $-CH_3$ | $C_6H_5$ | H | H | 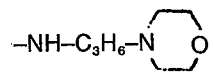 | Isopropanol | 153 | 50 | 35 |
| 217 | N | S | $-CH_3$ | $C_6H_5$ | H | H | $-NH-(CH_2)_5-CH_3$ | Ethanol | 112 | 75 | 35 |
| 218 | N | S | $-CH_3$ | $C_6H_5$ | H | H | 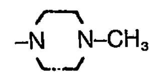 | Isopropanol | 167 | 65 | 35 |
| 219 | N | S | $-CH_3$ | $C_6H_5$ | H | CN | 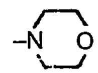 | Ethanol | 239 | 78 | 34 |
| 220 | N | NH | $-CH_3$ | $C_6H_5$ | H | H | 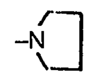 | DMF | ~280 | 25 | 36 |
| 221 | N | NH | $-CH_3$ | $C_6H_5$ | H | H | 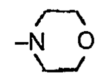 | DMF | ~280 | 30 | 36 |
| 222 | N | S | $-CH_3$ | $-CH_3$ | H | H | 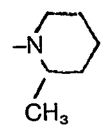 | Isopropanol | 149–51 × HCl | 18 | 35 |
| 223 | N | S | $-CH_3$ | 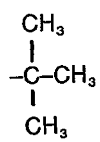 | H | H | 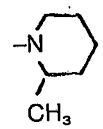 | Isopropanol | 173–75 × HCl | 23 | 35 |

Tabelle 3 (Fortsetzung)

| Beispiel Nr. | X | Y | R¹ | R² | R³ | R⁴ | R⁵ | Lösungsmittel (für Krist.) | Fp. °C | Ausbeute % d. Th. | analog Beispiel |
|---|---|---|---|---|---|---|---|---|---|---|---|
| 224 | N | S | $-CH_3$ | 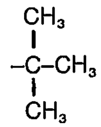 | H | H | 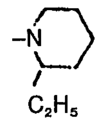 | Isopropanol | 180–82 × HCl | 37 | 35 |
| 225 | N | S | $-CH_3$ | $-CH_3$ | H | H | 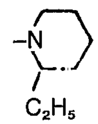 | Isopropanol | 139–41 × HCl | 42 | 35 |
| 226 | N | S | 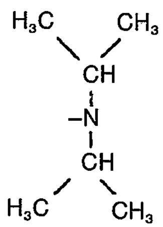 | $-C_6H_5$ | H | H | 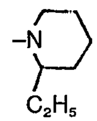 | Isopropanol | 133–35 × HCl | 52 | 35 |
| 227 | N | S | $-CH_3$ | $-CH_3$ | H | H | 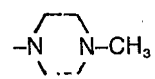 | Isopropanol | 300–02 × HCl | 37 | 35 |
| 228 | N | S | 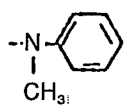 | $C_6H_5$ | H | H | 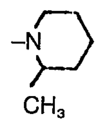 | Isopropanol | 158–60 × HCl | 47 | 35 |

Beispiel 229

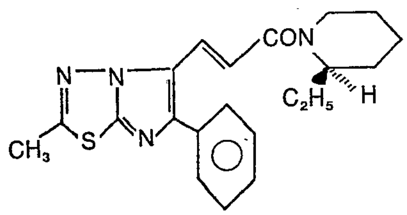

(−)-R-trans-N-[β-(2-methyl-6-phenyl-imidazo-[2,1-b]-1,3,4-thiadiazol-5-yl)-propenoyl]-2-ethyl-piperidin

5,7 g trans-β-(2-Methyl-6-phenyl-imidazo-[2,1-b]-1,3,4-thiadiazol-5-yl)-propensäure (Beispiel 18) werden in 60 ml Toluol mit 2 ml Thionylchlorid (Tropftrichter) bei 65 °C versetzt. Man kocht 2 Stunden unter Rückfluss (klare Lösung). Dann tropft man bei 40 °C 8 ml (−)-R-2-Ethylpiperidin (H. Frese, Ber. Dtsch. Chem. Ges. 33, 3483 (1900); H.C. Beyerman et al., Rec. Trac. Chim. Pays-Bas 90, 755 (1971)) zu und kocht weitere 60 Minuten unter Rückfluss. Man kühlt auf Raumtemperatur, saugt vom Niederschlag ab und extrahiert das Filtrat zweimal mit 30 ml Wasser. Die toluolische Lösung wird über Natriumsulfat getrocknet und am Rotationsverdampfer eingeengt. Der Rückstand (8,0 g) wird an einer Kieselgelsäule chromatographiert (Elution mit Chloroform). Die Fraktionen werden nach der Dünnschichtchromatographie zusammengefasst und aus Cyclohexan umkristallisiert.

Ausbeute: 7,2 g (94% der Theorie) vom Fp. 113–4 °C;

spez. Drehung (in $CHCl_3$) $[\alpha]^{20}_{589} = -34,89°$.

$C_{21}H_{24}N_4OS$ (380.15)

Ber. C 66.3 H 6,4 N 14,7 S 8,4

Gef. C 66,0 G 6,4 N 14,6 S 8,7

Beispiel 230

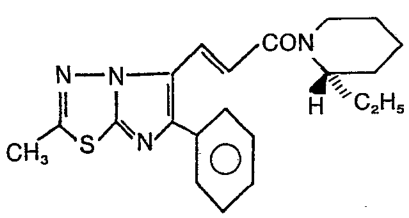

(+)-S-trans-N-[β-(2-methyl-6-phenyl-imidazo-[2,1-b]-1,3,4-thiadiazol-5-yl)-propenoyl]-2-ethyl-piperidin

8,7 g trans-β-(2-Methyl-6-phenyl-imidazol-[2,1-b]-1,3,4-thiadiazol-5-yl)-propensäure (Beispiel 18) werden in 90 ml Toluol mit 3 ml Thionylchlorid (Tropftrichter) bei 65 °C versetzt. Man kocht 2 Std. unter Rückfluss (klare Lösung). Dann tropft man bei 40 °C 12 g (+)-S-2-Ethylpiperidin (H. Frese, Ber. Dtsch. Chem. Ges. 33, 3483 (1900); H.C. Beyerman et al., Rec. Trac. Chim. Pays-Bas 90, 755 (1971)) zu und kocht weitere 60 Min. unter Rückfluss. Man kühlt auf Raumtemperatur, saugt vom Niederschlag ab und extrahiert das Filtrat dreimal mit 30 ml Wasser. Die toluolische Lösung wird über Natriumsulfat getrocknet und am Rotationsverdampfer eingeengt. Der Rückstand wird aus Cyclohexan umkristallisiert.

Ausbeute 5,0 g (44% d.Th.) vom Fp. 112–3 °C;

spez. Drehung (in $CHCl_3$) $[\alpha]^{20}_{589} = +33,02°$.

$C_{21}H_{24}N_4OS$ (380.15)

Ber. C 66,3 H 6,4 N 14,7 S 8,4

Gef. C 65,9 H 6,3 N 14,7 S 8,6

**Patentansprüche**

1. Imidazoazolalkensäureamide der allgemeinen Formel

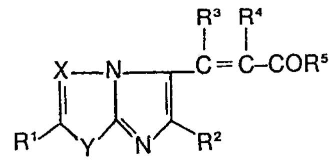

(I)

in welcher

X für N oder CH steht,

Y für S, O, NH oder N-Alkyl mit 1 bis 4 Kohlenstoffatomen steht,

$R^1$ und $R^2$ gleich oder verschieden sind und jeweils für Wasserstoff, geradkettiges oder verzweigtes oder cyclisches Alkyl, Alkenyl oder Alkinyl mit bis zu 6 Kohlenstoffatomen, welches gegebenenfalls substituiert ist durch Phenyl, Cyano, Hydroxyl, Methyl, Ethyl, Methoxy, Ethoxy, Trifluormethyl, Fluor, Chlor oder Brom stehen, wobei die Kohlenstoffkette des Alkylrestes gegebenenfalls unterbrochen ist durch O, S, NH, N-Alkyl mit 1 bis 4 Kohlenstoffatomen oder durch N-Benzyl,

oder

für einen Phenyl-, Naphthyl-, Furyl-, Thienyl-, Pyrimidyl-, Pyrazinyl-, Chinolyl-, Isochinolyl- oder Pyridylring stehen, der gegebenenfalls substituiert ist durch 1, 2 oder 3 gleiche oder verschiedene Substituenten aus der Gruppe Nitro, Trifluormethyl, Fluor, Chlor, Brom, Alkyl, Alkoxy oder $SO_n$-Alkyl (n = 0, 1 oder 2), wobei die genannten Alkyl- und Alkoxyreste jeweils 1 bis 4 Kohlenstoffatome enthalten,

für eine $SO_n$-Alkylgruppe stehen, wobei n und Alkyl die oben angegebene Bedeutung haben,

oder

für den Rest 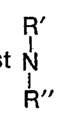

stehen, wobei R′ und R″ gleich oder verschieden sind und jeweils für Wasserstoff, Benzyl, Phenyl oder geradkettiges, verzweigtes oder cyclisches Alkyl mit 1 bis 6 Kohlenstoffatomen stehen, wobei die Alkylreste ihrerseits durch O, S oder N-Alkyl (1–4 C-Atome) unterbrochen sein können oder wobei R′ und R″ gemeinsam mit dem Stickstoffatom einen 5- bis 7-gliedrigen Ring bilden, der seinerseits gegebenenfalls 1 oder 2 weitere gleiche oder verschiedene Heteroatome aus der Gruppe Sauerstoff, Schwefel oder Stickstoff enthalten kann, wobei der Stickstoff gegebenenfalls durch Wasserstoff, Alkyl mit 1 bis 4 Kohlenstoffatomen, Phenyl oder Benzyl substituiert ist,

oder

für den Rest COR‴ stehen, wobei R‴ Wasserstoff,

Hydroxyl, Alkyl, Alkoxy mit jeweils 1 bis 6 C-Atomen, Phenoxy, Benzyloxy, Alkenoxy mit bis zu 4 C-Atomen, oder den Rest 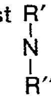

bedeutet, wobei R′ und R″ die oben angegebene Bedeutung haben,

$R^3$ für Wasserstoff, Trifluormethyl oder Alkyl mit 1 bis 4 Kohlenstoffatomen steht,

$R^4$ für Wasserstoff, Cyano, Nitro, Fluor, Chlor, Brom oder $SO_n$-Alkyl (1–4 C-Atome; n = 0, 1 oder 2) steht, oder für Alkoxy mit 1 bis 4 C-Atomen steht,

oder

für geradkettiges, verzweigtes oder cyclisches Alkyl, Alkenyl oder Alkinyl mit jeweils bis zu 6 C-Atomen steht, wobei der Alkylrest gegebenenfalls substituiert ist durch Fluor, Chlor, Brom oder Cyano und gegebenenfalls unterbrochen ist durch O, S, NH, N-Benzyl oder N-Alkyl (1–4 C-Atome) oder für den Rest COR‴ steht, wobei R‴ die oben in der Definition von $R^1$ und $R^2$ angegebene Bedeutung besitzt,

oder

und mit diesem identisch oder verschieden sein kann, und

$R^5$ für eine Aminogruppe der Formel 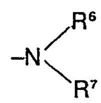 steht,

in welcher

a) $R^6$ Wasserstoff, Phenyl oder einen geradkettigen, verzweigten, cyclischen, gesättigten oder ungesättigten aliphatischen Kohlenwasserstoffrest mit bis zu 10 Kohlenstoffatomen bedeutet, der gegebenenfalls durch O, S, N, N-Alkyl (1–4 C-Atome), NH, N-Phenyl oder N-Benzyl unterbrochen ist und der gegebenenfalls substituiert ist durch Hydroxy, Alkoxy, Alkyl, Fluor, Chlor, Brom, Phenyl, Alkoxycarbonyl oder Dialkylamino mit jeweils 1 bis 4 C-Atomen in den Alkyl- und Alkoxyresten, wobei die beiden Alkylreste gegebenenfalls mit dem N-Atom einen 5- bis 7-gliedrigen Ring bilden, der gegebenenfalls durch O, S, NH oder N-Alkyl (1 bis 4 C-Atome) unterbrochen ist, und wobei die vorgenannten Alkyl- und Phenylreste ihrerseits gegebenenfalls substituiert sind durch Fluor, Chlor, Brom, Trifluormethyl, Phenyl, Benzyl, Alkyl, Alkoxy oder $SO_2$-Alkyl, mit jeweils 1 bis 4 C-Atomen in den Alkyl- und Alkoxyresten oder

b) $R^6$ und $R^7$ gemeinsam mit dem Stickstoffatom einen 4- bis 7-gliedrigen Ring bilden, der gegebenenfalls 1 oder 2 weitere Heteroatome aus der Gruppe Sauerstoff, Schwefel oder Stickstoff enthält, wobei der Stickstoff gegebenenfalls durch Wasserstoff, Alkyl (1–4 C-Atome), Phenyl oder Benzyl substituiert ist und wobei dieser 4- bis 7-gliedrige Ring durch 1 bis 4 gleiche oder verschiedene Substituenten aus der Gruppe Halogen, Trifluormethyl, Phenyl, Benzyl, Alkyl, Hydroxyalkyl, Alkoxyalkyl oder Alkoxycarbonyl mit jeweils 1 bis 4 C-Atomen in den Alkyl- und Alkoxyresten substituiert sein kann, oder wobei dieser Ring mit einem gegebenenfalls durch Fluor, Chlor, Brom, Nitro oder Hydroxy substituierten aromatischen Ring kondensiert sein kann,

c) $R^7$ die unter a) angegebene Bedeutung von $R^6$ besitzt, wobei $R^7$ und $R^6$ gleich oder verschieden sein können,

oder

d) einer der beiden Reste $R^6$ oder $R^7$ für die Gruppe 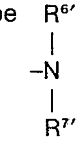 ,

steht, wobei die Reste $R^{6'}$ und $R^{7'}$ die unter a) und b) angegebene Bedeutung von $R^6$ und $R^7$ haben (Hydrazine)

und ihre pharmazeutisch unbedenklichen Säureadditionssalze sowie ihre stereoisomeren Formen.

2. Verbindungen der allgemeinen Formel (I) gemäss Anspruch 1, in welcher

X für N oder CH steht,

Y für S, O oder NH steht,

$R^1$ und $R^2$ gleich oder verschieden sind und jeweils für Wasserstoff, Trifluormethyl, Phenyl, Alkyl oder Alkenyl mit bis zu 6 Kohlenstoffatomen, gegebenenfalls substituiert durch Fluor, Chlor, Hydroxyl, Trifluormethyl, Alkyl oder Alkoxy mit je 1 bis 2 Kohlenstoffatomen, oder für Pyridyl, Thienyl, Furyl, Naphthyl, Pyrimidyl, Pyrazinyl, Chinolyl oder Isochinolyl stehen,

oder

für den Rest 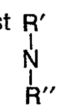

stehen, wobei R′ und R″ gleich oder verschieden sind und jeweils für Wasserstoff, Benzyl, Phenyl oder geradkettiges, verzweigtes oder cyclisches Alkyl mit bis zu 6 Kohlenstoffatomen stehen, wobei die Alkylreste ihrerseits durch O, S oder N-Alkyl (1–2 C-Atome) unterbrochen sein können oder wobei R′ und R″ gemeinsam mit dem Stickstoffatom einen 5- bis 7-gliedrigen Ring bilden, der seinerseits 1 oder 2 gleiche oder verschiedene Heteroatome aus der Gruppe Sauerstoff, Schwefel oder Stickstoff enthalten kann, wobei der Stickstoff gegebenenfalls durch Wasserstoff, Alkyl mit 1 bis 2 Kohlenstoffatomen, Phenyl oder Benzyl substituiert ist,

oder

für den Rest COR‴ stehen, wobei R‴ Wasserstoff, Hydroxyl, Alkyl, Alkoxy mit jeweils bis zu 4 Kohlenstoffatomen, Phenoxy, Benzyloxy oder den Rest 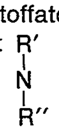

bedeutet, wobei R′ und R″ die oben angegebene Bedeutung haben,

$R^3$ für Wasserstoff, Trifluormethyl oder Alkyl mit 1 bis 4 Kohlenstoffatomen steht,

$R^4$ für Wasserstoff, Alkyl oder Alkoxy mit jeweils 1 bis 4 Kohlenstoffatomen, gegebenenfalls unterbrochen durch Sauerstoff und gegebenenfalls substituiert durch Fluor, Chlor oder Cyano steht,

oder

für Cyano, Nitro oder für den Rest COR‴ steht,

wobei R''' die oben in der Definition von $R^1$ und $R^2$ angegebene Bedeutung besitzt,

oder

für $SO_n$-Alkyl (n = 0 oder 2) mit 1 bis 4 Kohlenstoffatomen im Alkylteil steht, und

$R^5$ für eine Aminogruppe der Formel

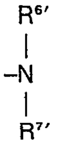

,

steht,

in welcher

a) $R^6$ Wasserstoff, Phenyl oder einen geradkettigen, verzweigten, cyclischen, gesättigten oder ungesättigten Kohlenwasserstoffrest mit bis zu 10 Kohlenstoffatomen bedeutet, der gegebenenfalls durch O, S, N, NH, N-Phenyl oder N-Benzyl unterbrochen ist und der gegebenenfalls substituiert ist durch Hydroxy, Alkoxy, Fluor, Chlor, Brom, Phenyl, Alkoxycarbonyl oder Dialkylamino mit jeweils 1 bis 4 C-Atomen in den Alkyl- und Alkoxyresten, wobei die beiden Alkylreste gegebenenfalls mit dem N-Atom einen 5- oder 6-gliedrigen Ring bilden, der gegebenenfalls durch O, S oder NH unterbrochen ist, und wobei die vorgenannten Alkyl- und Phenylreste ihrerseits gegebenenfalls substituiert sind durch Fluor, Chlor, Brom, Trifluormethyl, Alkyl, Alkoxy oder $SO_2$-Alkyl, mit jeweils 1 bis 4 C-Atomen in den Alkyl- und Alkoxyresten,

oder

b) $R^6$ und $R^7$ gemeinsam mit dem Stickstoffatom einen 4- bis 7-gliedrigen Ring bilden, der gegebenenfalls 1 oder 2 weitere Heteroatome aus der Gruppe Sauerstoff, Schwefel oder Stickstoff enthält, wobei der Stickstoff gegebenenfalls durch Wasserstoff, Alkyl (1–2 C-Atome), Phenyl oder Benzyl substituiert ist und wobei dieser 4- bis 7-gliedrige Ring durch 1 bis 4 gleiche oder verschiedene Substituenten aus der Gruppe Fluor, Chlor, Trifluormethyl, Alkyl, Hydroxyalkyl, Alkoxyalkyl oder Alkoxycarbonyl mit jeweils 1 bis 4 C-Atomen in den Alkyl- und Alkoxyresten substituiert sein kann, oder

wobei dieser Ring mit einem gegebenenfalls durch Fluor, Chlor, Nitro oder Hydroxy substituierten aromatischen Ring kondensiert sein kann,

c) $R^7$ die unter a) angegebene Bedeutung von $R^6$ besitzt, wobei $R^7$ und $R^6$ gleich oder verschieden sein können

oder

d) einer der beiden Reste $R^6$ oder $R^7$ für die Gruppe

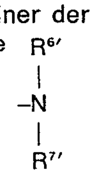

,

steht, wobei die Reste $R^{6'}$ und $R^{7'}$ die unter a) und b) angegebene Bedeutung von $R^6$ und $R^7$ haben (Hydrazine)

und ihre pharmazeutisch unbedenklichen Säureadditionssalze, sowie ihre stereoisomeren Formen.

3. Verbindung der Formel

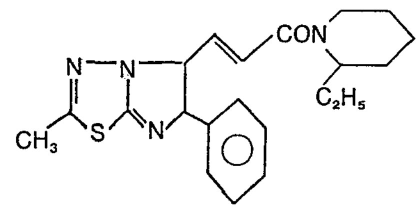

4. Verbindung der Formel

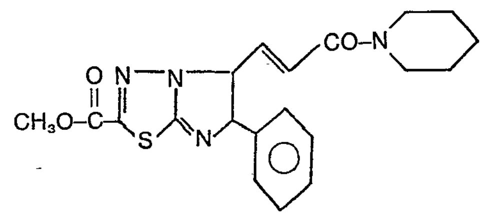

5. Verfahren zur Herstellung von Verbindungen der allgemeinen Formel (I) gemäss Anspruch 1, dadurch gekennzichnet, dass man

a) Carbonylverbindungen der allgemeinen Formel (II)

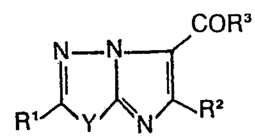

(II)

in welcher

X, Y, $R^1$, $R^2$ und $R^3$ die in Anspruch 1 angegebene Bedeutung haben, mit Phosphonatverbindungen der allgemeinen Formel (III)

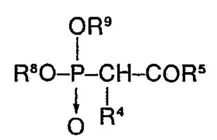

(III)

in welcher

$R^4$ und $R^5$ die in Anspruch 1 angegebene Bedeutung haben, und

$R^8$ und $R^9$ für gegebenenfalls substituiertes Alkyl oder Aralkyl stehen,

in Gegenwart von starken Basen und inerten organischen Lösungsmitteln bei Temperaturen zwischen −20 °C und +40 °C umsetzt oder

b) Carbonylverbindungen der allgemeinen Formel (II) mit Acetamidderivaten der allgemeinen Formel (IV)

$$R^4\text{–}CH_2\text{–}COR^5 \qquad (IV)$$

in welcher

$R^4$ und $R^5$ die in Anspruch 1 angegebene Bedeutung haben,

in Gegenwart von sauren oder basischen Katalysatoren und gegebenenfalls in Gegenwart von inerten organischen Lösungsmitteln bei Temperaturen zwischen 20 und 200 °C umsetzt

oder

c) Alkensäuren der allgemeinen Formel (V)

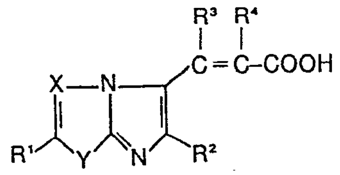

(V)

in welcher

X, Y, $R^1$, $R^2$, $R^3$ und $R^4$ die in Anspruch 1 angegebene Bedeutung haben,

mit Aminen der allgemeinen Formel (VI)

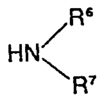

(VI)

in welcher

$R^6$ und $R^7$ die oben angegebene Bedeutung haben,

gegebenenfalls nach Aktivierung der Carboxylgruppe über das entsprechende Säurehalogenid in üblicher Weise, gegebenenfalls in Gegenwart von inerten organischen Lösungsmitteln bei Temperaturen zwischen 20 und 150 °C amidiert.

6. Alkensäuren der allgemeinen Formel (V)

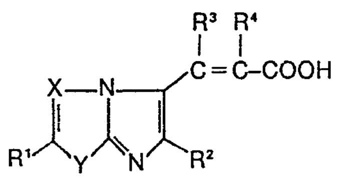

(V)

in welcher

X, Y, $R^1$, $R^2$, $R^3$ und $R^4$ die im Anspruch 1 angegebene Bedeutung besitzen.

7. Arzneimittel, enthaltend mindestens eine Verbindung gemäss Anspruch 1 bis 4.

8. Verbindungen gemäss Anspruch 1 bis 4 zur Verwendung bei der Bekämpfung von Erkrankungen.

9. Verfahren zur Herstellung von Alkensäuren der allgemeinen Formel (V)

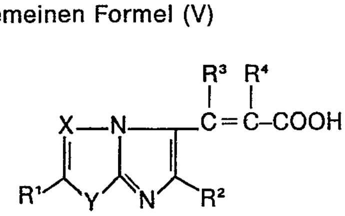

(V)

in welcher

X, Y, $R^1$, $R^2$, $R^3$ und $R^4$ die im Anspruch 1 angegebene Bedeutung haben,

dadurch gekennzeichnet, dass man

a) Carbonylverbindungen der allgemeinen Formel (II)

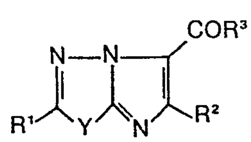

(II)

in welcher

X, Y, $R^1$ $R^2$ und $R^3$ die oben angegebene Bedeutung haben,

mit Phosphonatverbindungen der allgemeinen Formel (VII)

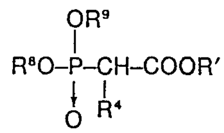

(VII)

in welcher

$R^4$, $R^8$ und $R^9$ die in Anspruch 5 angegebene Bedeutung besitzen und

R′ für gegebenenfalls substituiertes Alkyl oder Aralkyl steht,

zu Alkensäureestern umsetzt und diese dann in Gegenwart von Basen zu den entsprechenden Alkensäuren der allgemeinen Formel (V) verseift oder

b) für den Fall, dass $R^3$ Wasserstoff bedeutet, Aldehyde der allgemeinen Formel (VIII)

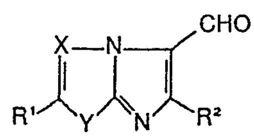

(VIII)

in welcher

X, Y, $R^1$ und $R^2$ die oben angegebene Bedeutung haben,

mit Malonsäuren der allgemeinen Formel (IX)

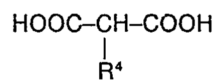

(IX)

in welcher

$R^4$ die oben angegebene Bedeutung hat,

in Gegenwart von inerten organischen Lösungsmitteln und gegebenenfalls in Gegenwart von Kondensationsmitteln kondensiert.

**Claims**

1. Imidazoazole alkenoic acid amides of the general formula

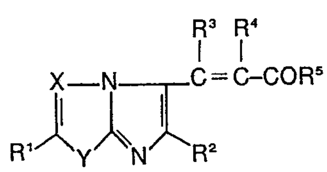

(I)

in which

X represents N or CH,

Y represents S, O, NH or N-alkyl with 1 to 4 carbon atoms,

$R^1$ and $R^2$ are identical or different and each represent hydrogen, straight-chain or branched or cyclic alkyl, alkenyl or alkinyl with up to 6 carbon atoms, which is optionally substituted by phenyl, cyano, hydroxyl, methyl, ethyl, methoxy, ethoxy, trifluoromethyl, fluorine, chlorine or bromine, the carbon chain of the alkyl radical optionally being interrupted by O, S, NH, N-alkyl with 1 to 4 carbon atoms or by N-benzyl, or represent a phenyl, naphthyl, furyl, thienyl, pyrimidyl, pyrazinyl, quinolyl, isoquinolyl or pyridyl ring which is optionally substituted by 1, 2 or 3 identical or different substituents from the group comprising nitro, trifluoromethyl, fluorine, chlorine, bromine, alkyl, alkoxy or $SO^n$-alkyl (n = 0, 1 or 2), the alkyl and alkoxy radicals mentioned each containing 1 to 4 carbon atoms,

or represent a $SO_n$-alkyl group, n and alkyl having the meaning given above,

or represent the radical 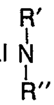

wherein R′ and R″ are identical or different and each represent hydrogen, benzyl, phenyl or straight-chain, branched or cyclic alkyl with 1 to 6 carbon atoms, it being possible for the alkyl radicals in turn to be interrupted by O, S or N-alkyl (1–4 C atoms), or wherein R′ and R″, together with the nitrogen atom, form a 5- to 7-membered ring which in turn can optionally contain 1 or 2 further identical or different hetero atoms from the group comprising oxygen, sulphur or nitrogen, the nitrogen atom optionally being substituted by hydrogen, alkyl with 1 to 4 carbon atoms, phenyl or benzyl,

or represent the radical COR‴, wherein R‴ denotes hydrogen, hydroxyl, alkyl or alkoxy with in each case 1 to 6 C atoms, phenoxy, benzyloxy, alkenoxy with up to 4 C atoms or the radical 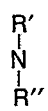

wherein R′ and R″ have the meaning given above,

$R^3$ represents hydrogen, trifluoromethyl or alkyl with 1 to 4 carbon atoms,

$R^4$ represents hydrogen, cyano, nitro, fluorine, chlorine, bromine or $SO_n$-alkyl (1–4 C-atoms; n = 0, 1 or 2), or represents alkoxy with 1 to 4 C atoms,

or represents straight-chain, branched or cyclic alkyl, alkenyl or alkinyl with in each case up to 6 C atoms, the alkyl radical optionally being substituted by fluorine, chlorine, bromine or cyano and optionally being interrupted by O, S, NH, N-benzyl or N-alkyl (1–4 C atoms),

or represents the radical COR‴, wherein R‴ has the meaning given above in the definition of $R^1$ and $R^2$,

or

and can be identical to or different from the latter, and

$R^5$ represents an amino group of the formula

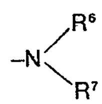

in which

a) $R^6$ denotes hydrogen, phenyl or a straight-chain, branched, cyclic, saturated or unsaturated aliphatic hydrocarbon radical with up to 10 carbon atoms, which is optionally interrupted by O, S, N, N-alkyl (1–4 C atoms), NH, N-phenyl or N-benzyl and which is optionally substituted by hydroxyl, alkoxy, alkyl, fluorine, chlorine, bromine, phenyl, alkoxycarbonyl or dialkylamino with in each case 1 to 4 C atoms in the alkyl and alkoxy radicals, the two alkyl radicals optionally forming a 5- to 7-membered ring with the N-atom, which ring is optionally interrupted by O, S, NH or N-alkyl (1 to 4 C atoms), and the aforementioned alkyl and phenyl radicals in turn being optionally substituted by fluorine, chlorine, bromine, trifluoromethyl, phenyl, benzyl, alkyl, alkoxy or $SO_2$-alkyl, with in each case 1 to 4 C atoms in the alkyl and alkoxy radicals or

b) $R^6$ and $R^7$, together with the nitrogen atom, form a 4- to 7-membered ring which optionally contains 1 or 2 further hetero atoms from the group comprising oxygen, sulphur or nitrogen, the nitrogen atom optionally being substituted by hydrogen, alkyl (1–4 C atoms), phenyl or benzyl and it being possible for this 4- to 7-membered ring to be substituted by 1 to 4 identical or different substituents from the group comprising halogen, trifluoromethyl, phenyl, benzyl, alkyl, hydroxyalkyl, alkoxyalkyl or alkoxycarbonyl with in each case 1 to 4 C atoms in the alkyl and alkoxy radicals, or it being possible for this ring to be fused to an aromatic ring which is optionally substituted by fluorine, chlorine, bromine, nitro or hydroxyl,

c) $R^7$ has the meaning of $R^6$ given under a), it being possible for $R^7$ and $R^6$ to be identical or different,

or

d) one of the two radicals $R^6$ or $R^7$ represents the group 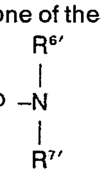 ,

the radicals $R^{6'}$ and $R^{7'}$ having the meaning of $R^6$ and $R^7$ given under a) and b) (hydrazines),

and their pharmaceutically harmless acid addition salts and their stereoisomeric forms.

2. Compounds of the general formula (I) according to Claim 1, in which

X represents N or CH,

Y represents S, O or NH,

$R^1$ and $R^2$ are identical or different and each represent hydrogen, trifluoromethyl, phenyl, alkyl or alkenyl with up to 6 carbon atoms, optionally substituted by fluorine, chlorine, hydroxyl, trifluoromethyl, or alkyl or alkoxy each with 1 to 2 carbon atoms, or pyridyl, thienyl, furyl, naphthyl, pyrimidyl, pyrazinyl, quinolyl or isoquinolyl,

or

represent the radical 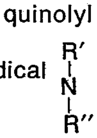

wherein R′ and R″ are identical or different and each represent hydrogen, benzyl, phenyl or straight-chain, branched or cyclic alkyl with up to 6 carbon atoms, it being possible for the alkyl radicals in turn to be interrupted by O, S or N-alkyl (1–2 C atoms) or wherein R′ and R″, together with the nitrogen atom, form a 5- to 7-membered ring which can in turn contain 1 or 2 identical or different hetero atoms from the group comprising

oxygen, sulphur or nitrogen, the nitrogen atom optionally being substituted by hydrogen, alkyl with 1 to 2 carbon atoms, phenyl or benzyl, or represent the radical COR‴, wherein R‴ denotes hydrogen, hydroxyl, alkyl or alkoxy with in each case up to 4 carbon atoms, phenoxy, benzyloxy or the radical

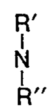

wherein R′ and R″ have the meaning given above,

$R^3$ represents hydrogen, trifluoromethyl or alkyl with 1 to 4 carbon atoms,

$R^4$ represents hydrogen, alkyl or alkoxy with in each case 1 to 4 carbon atoms, optionally interrupted by oxygen and optionally substituted by fluorine, chlorine or cyano, or represents cyano, nitro or the radical COR‴, wherein R‴ has the meaning given above in the definition of $R^1$ and $R^2$, or represents $SO_n$-alkyl (n = 0 or 2) with 1 to 4 carbon atoms in the alkyl part, and

$R^5$ represents an amino group of the formula

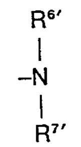

in which

a) $R^6$ denotes hydrogen, phenyl or a straight-chain, branched, cyclic, saturated or unsaturated hydrocarbon radical with up to 10 carbon atoms, which is optionally interrupted by O, S, N, NH, N-phenyl or N-benzyl and which is optionally substituted by hydroxyl, alkoxy, fluorine, chlorine, bromine, phenyl, alkoxycarbonyl or dialkylamino with in each case 1 to 4 C atoms in the alkyl and alkoxy radicals, the two alkyl radicals optionally forming a 5- or 6-membered ring with the N-atom, which ring is optionally interrupted by O, S, or NH, and the aforementioned alkyl and phenyl radicals in turn being optionally substituted by fluorine, chlorine, bromine, trifluoromethyl, alkyl, alkoxy or $SO_2$-alkyl, with in each case 1 to 4 C atoms in the alkyl and alkoxy radicals,

or

b) $R^6$ and $R^7$, together with the nitrogen atom, form a 4- to 7-membered ring which optionally contains 1 or 2 further hetero atoms from the group comprising oxygen, sulphur or nitrogen, the nitrogen atom being optionally substituted by hydrogen, alkyl (1–2 C atoms), phenyl or benzyl, and it being possible for this 4- to 7-membered ring to be substituted by 1 to 4 identical or different substituents from the group comprising fluorine, chlorine, trifluoromethyl, alkyl, hydroxyalkyl, alkoxyalkyl or alkoxycarbonyl with in each case 1 to 4 C atoms in the alkyl and alkoxy radicals, or it being possible for this ring to be fused to an aromatic ring which is optionally substituted by fluorine, chlorine, nitro or hydroxyl,

c) $R^7$ has the meaning of $R^6$ given under a), it being possible for $R^7$ and $R^6$ to be identical or different or

d) one of the two radicals $R^6$ or $R^7$ represents the group

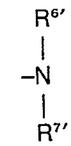

wherein the radicals $R^{6'}$ and $R^{7'}$ have the meaning of $R^6$ and $R^7$ given under a) and b) (hydrazines), and their pharmaceutically harmless acid addition salts and their stereoisomeric forms.

3. Compound of the formula

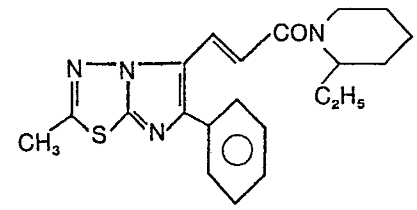

4. Compound of the formula

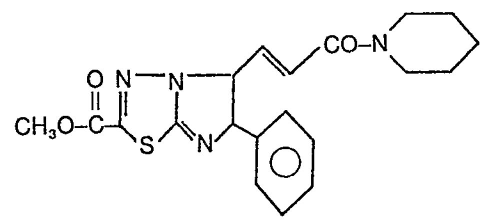

5. Process for the preparation of compounds of the general formula (I) according to Claim 1, characterised in that

a) carbonyl compounds of the general formula (II)

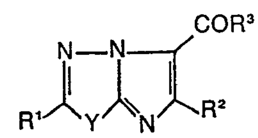

(II)

in which

X, Y, $R^1$, $R^2$ and $R^3$ have the meaning given in Claim 1,

are reacted with phosphonate compounds of the general formula (III)

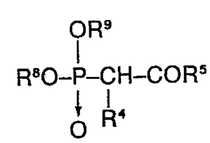

(III)

in which

$R^4$ and $R^5$ have the meaning given in Claim 1 and

$R^8$ and $R^9$ represent optionally substituted alkyl or aralkyl,

in the presence of strong bases and inert organic solvents at temperatures between −20 °C and +40 °C or

b) carbonyl compounds of the general formula (II) are reacted with acetamide derivatives of the general formula (IV)

$R^4$–$CH_2$–$COR^5$ (IV)

in which

$R^4$ and $R^5$ have the meaning given in Claim 1, in the presence of acid or basic catalysts and if appropriate in the presence of inert organic solvents at temperatures between 20 and 200 °C or

c) alkenoic acids of the general formula (V)

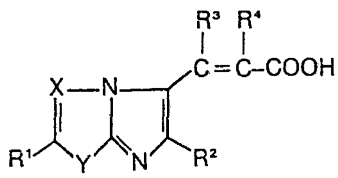
(V)

in which

X, Y, $R^1$, $R^2$, $R^3$ and $R^4$ have the meaning given in Claim 1,

are amidated with amines of the general formula (VI)

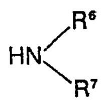
(VI)

in which

$R^6$ and $R^7$ have the meaning given above,

if appropriate after activating the carboxyl group via the corresponding acid halide, in the customary manner, if appropriate in the presence of inert organic solvents, at temperatures between 20 and 150 °C.

6. Alkenoic acids of the general formula (V)

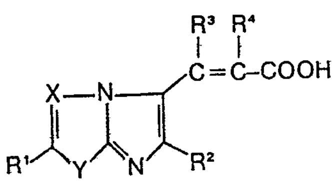
(V)

in which

X, Y, $R^1$, $R^2$, $R^3$ and $R^4$ have the meaning given in Claim 1.

7. Medicaments containing at least one compound according to Claim 1 to 4.

8. Compounds according to Claim 1 to 4 for use in combating diseases.

9. Process for the preparation of alkenoic acids of the general formula (V)

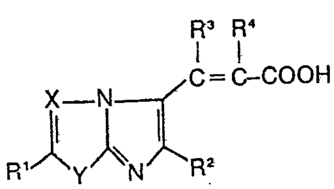
(V)

in which

X, Y, $R^1$, $R^2$, $R^3$ and $R^4$ have the meaning given in Claim 1, characterised in that

a) carbonyl compounds of the general formula (II)

in which

X, Y, $R^1$, $R^2$ and $R^3$ have the meaning given above,

are reacted with phosphonate compounds of the general formula (VII)

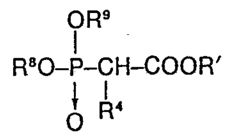
(VII)

in which

$R^4$, $R^8$ and $R^9$ have the meaning given in Claim 5 and

R′ represents optionally substituted alkyl or aralkyl, to give alkenoic acid esters and these are then saponified in the presence of bases to give the corresponding alkenoic acids of the general formula (V),

or

b) in the case where $R^3$ denotes hydrogen, aldehydes of the general formula (VIII)

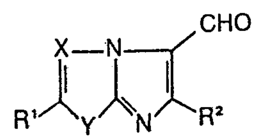
(VIII)

in which

X, Y, $R^1$ and $R^2$ have the meaning given above, are subjected to a condensation reaction with malonic acids of the general formula (IX)

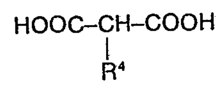
(IX)

in which

$R^4$ has the meaning given above,

in the presence of inert organic solvents and if appropriate in the presence of condensing agents.

**Revendications**

1. Amides d'acides imidazo-alcénoïques, de formule générale

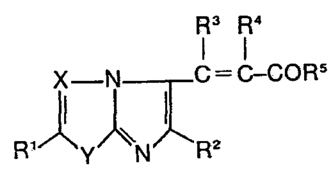
(I)

dans laquelle

X représente N ou CH,

Y représente S, O, NH ou un groupe N-alkyle ayant 1 à 4 atomes de carbone,

$R^1$ et $R^2$ sont identiques ou différents et représentent chacun l'hydrogène, un groupe alkyle, alcényle ou alcynyle à chaîne droite ou ramifiée ou cyclique ayant jusqu'à 6 atomes de carbone, qui est éventuellement substitué par un substituant phényle, cyano, hydroxyle, méthyle, éthyle, méthoxy, éthoxy, trifluorométhyle, fluoro, chloro

ou bromo, la chaîne carbonée du reste alkyle étant éventuellement interrompue par O, S, NH, un groupe N-alkyle ayant 1 à 4 atomes de carbone ou un groupe N-benzyle,

ou bien

un noyau phényle, naphtyle, furyle, thiényle, pyrimidyle, pyrazinyle, quinolyle, isoquinolyle ou pyridyle qui est éventuellement substitué par 1, 2 ou 3 substituants identiques ou différents du groupe des substituants nitro, trifluorométhyle, fluoro, chloro, bromo, alkyle, alkoxy ou $SO_n$-alkyle ($n = 0, 1$ ou 2), les restes alkyle et alkoxy mentionnés contenant chacun 1 à 4 atomes de carbone, un groupe $SO_n$-alkyle, n et alkyle ayant les définitions indiquées ci-dessus, ou le reste 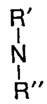

R′ et R″ étant identiques ou différents et représentant chacun l'hydrogène ou un groupe benzyle, phényle ou alkyle à chaîne droite, ramifiée ou cyclique ayant 1 à 6 atomes de carbone, les restes alkyle pouvant être interrompus quant à eux par O, S ou un groupe N-alkyle (1 à 4 atomes de carbone) ou bien R′ et R″ forment conjointement avec l'atome d'azote un noyau pentagonal à heptagonal qui peut comporter quant à lui éventuellement 1 ou 2 autres hétéroatomes égaux ou différents du groupe des atomes d'oxygène, de soufre ou d'azote, l'azote étant éventuellement substitué par de l'hydrogène, un radical alkyle ayant 1 à 4 atomes de carbone, phényle ou benzyle,

ou bien

le reste COR‴, dans lequel R‴ représente l'hydrogène, un groupe hydroxyle, alkyle, alkoxy ayant chacun 1 à 6 atomes de carbone, phénoxy, benzyloxy, alcénoxy ayant jusqu'à 4 atomes de carbone, ou le reste 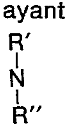

R′ et R″ ayant la définition indiquée ci-dessus,

$R^3$ désigne l'hydrogène, un groupe trifluorométhyle ou un groupe alkyle ayant 1 à 4 atomes de carbone,

$R^4$ désigne l'hydrogène, un groupe cyano ou nitro, un atome de fluor, de chlore, de brome ou un groupe $SO_n$-alkyle (1–4 atomes de carbone; $n = 0, 1$ ou 2) ou un groupe alkoxy ayant 1 à 4 atomes de carbone,

ou bien

un groupe alkyle, alcényle ou alcynyle à chaîne droite, ramifiée ou cyclique ayant dans chaque cas jusqu'à 6 atomes de carbone, le reste alkyle étant éventuellement substitué par du fluor, du chlore, du brome ou un radical cyano et étant éventuellement interrompu par O, S, NH, un groupe N-benzyle ou un groupe N-alkyle (1 à 4 atomes de carbone) ou le reste COR‴, dans lequel R‴ a la valeur indiquée ci-dessus dans la définition de $R^1$ et $R^2$,

ou bien

et peut y être identique ou en être différent, et

$R^5$ désigne un groupe amino de formule

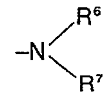

dans laquelle

a) $R^6$ est l'hydrogène, le groupe phényle ou un reste d'hydrocarbure aliphatique à chaîne droite, ramifiée, cyclique, saturé ou insaturé ayant jusqu'à 10 atomes de carbone, qui est éventuellement interrompu par O, S, N, N-alkyle (1–4 atomes de carbone), NH, N-phényle ou N-benzyle et qui est éventuellement substitué par un radical hydroxy, alkoxy, alkyle, fluoro, chloro, bromo, phényle, alkoxycarbonyle ou dialkylamino ayant chacun 1 à 4 atomes de carbone dans les restes alkyle et alkoxy, les deux restes alkyle formant éventuellement avec l'atome d'azote un noyau pentagonal à heptagonal qui est interrompu le cas échéant par O, S, NH ou un groupe N-alkyle (1 à 4 atomes de carbone), les restes alkyle et phényle mentionnés ci-dessus étant éventuellement substitués quant à eux par du fluor, du chlore, du brome, un groupe trifluorométhyle, phényle, benzyle, alkyle, alkoxy ou $SO_2$-alkyle avec dans chaque cas 1 à 4 atomes de carbone dans les restes alkyle et alkoxy, ou bien

b) $R^6$ et $R^7$ forment conjointement avec l'atome d'azote un noyau tétragonal à heptagonal qui comprend éventuellement 1 ou 2 autres hétéroatomes du groupe oxygène, soufre ou azote, l'azote étant éventuellement substitué par de l'hydrogène ou un radical alkyle (1 à 4 atomes de carbone), phényle ou benzyle et ce noyau tétragonal à heptagonal pouvant être substitué par 1 à 4 substituants identiques ou différents du groupe comprenant un halogène et des radicaux trifluorométhyle, phényle, benzyle, alkyle, hydroxyalkyle, alkoxyalkyle ou alkoxycarbonyle ayant dans chaque cas 1 à 4 atomes de carbone dans les restes alkyle et alkoxy, ou bien ce noyau peut être condensé avec un noyau aromatique éventuellement substitué par du fluor, du chlore, du brome, un substituant nitro ou hydroxy,

c) $R^7$ possède la définition indiquée en a) pour $R^6$, $R^7$ et $R^6$ pouvant être identiques ou différents, ou

d) l'un des deux restes $R^6$ ou $R^7$ représente le groupe 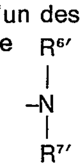

les restes $R^{6'}$ et $R^{7'}$ ayant la définition indiquée en a) et b) pour $R^6$ et $R^7$ (hydrazines)

et leurs sels d'addition d'acides pharmaceutiquement acceptables ainsi que leurs formes stéréoisomériques.

2. Composés de formule générale (I) suivant la revendication 1, dans laquelle

X représente N ou CH,

Y représente S, O ou NH,

$R^1$ et $R^2$ sont identiques ou différents et représentent chacun l'hydrogène, un groupe trifluorométhyle, phényle, alkyle ou alcényle ayant jusqu'à 6 atomes de carbone, éventuellement substitué

par du fluor, du chlore, un radical hydroxyle, trifluorométhyle, alkyle ou alkoxy ayant chacun 1 ou 2 atomes de carbone ou un groupe pyridyle, thiényle, furyle, naphtyle, pyrimidyle, pyrazinyle, quinolyle ou isoquinolyle,
ou bien
le reste R'
|
N
|
R''

R' et R'' étant identiques ou différents et représentant chacun l'hydrogène ou un groupe benzyle, phényle ou alkyle à chaîne droite, ramifiée ou cyclique, ayant jusqu'à 6 atomes de carbone, les restes alkyle pouvant être interrompus quant à eux par O, S ou un groupe N-alkyle (1 ou 2 atomes de carbone) ou bien R' et R'' peuvent former conjointement avec l'atome d'azote un noyau pentagonal à heptagonal qui peut comporter, quant à lui, 1 ou 2 hétéroatomes égaux ou différents choisis dans le groupe de l'oxygène, du soufre ou de l'azote, l'azote étant éventuellement substitué par de l'hydrogène, un radical alkyle ayant 1 ou 2 atomes de carbone, phényle ou benzyle, ou le reste COR''', R''' représentant l'hydrogène, un groupe hydroxyle, alkyle, alkoxy ayant chacun jusqu'à 4 atomes de carbone, phénoxy, benzyloxy ou le reste R'
|
N
|
R''

R' et R'' ayant les définitions indiquées ci-dessus,

$R^3$ représente l'hydrogène, le groupe trifluorométhyle ou un groupe alkyle ayant 1 à 4 atomes de carbone,

$R^4$ représente l'hydrogène, un groupe alkyle ou alkoxy ayant chacun 1 à 4 atomes de carbone, éventuellement interrompu par de l'oxygène et substitué le cas échéant par du fluor, du chlore ou un substituant cyano, ou
un groupe cyano, nitro ou le reste COR''' dans lequel R''' a la valeur indiquée ci-dessus dans la définition de $R^1$ et $R^2$, ou
un groupe $SO_n$-alkyle (n = 0 ou 2) ayant 1 à 4 atomes de carbone dans la partie alkyle, et

$R^5$ est un groupe amino de formule $-N(R^{6'})(R^{7'})$

dans laquelle

a) $R^6$ représente l'hydrogène, un groupe phényle ou un reste hydrocarboné à chaîne droite, ramifiée, cyclique, saturé ou insaturé ayant jusqu'à 10 atomes de carbone, qui est interrompu le cas échéant par O, S, N, NH, N-phényle ou N-benzyle et qui est éventuellement substitué par un substituant hydroxy, alkoxy, fluoro, chloro, bromo, phényle, alkoxycarbonyle ou dialkylamino ayant chacun 1 à 4 atomes de carbone dans les restes alkyle et alkoxy, les deux restes alkyle formant éventuellement avec l'atome d'azote un noyau pentagonal ou hexagonal qui est interrompu le cas échéant par O, S ou NH, et les restes alkyle et phényle mentionnés ci-dessus étant éventuellement substitués, quant à eux, par du fluor, du chlore, du brome, un substituant trifluorométhyle, alkyle alkoxy ou ou $SO_2$-alkyle ayant dans chaque cas 1 à 4 atomes de carbone dans les restes alkyle et alkoxy,
ou

b) $R^6$ et $R^7$ forment conjointement avec l'atome d'azote un noyau tétragonal à heptagonal qui comprend éventuellement 1 ou 2 autres hétéroatomes du groupe formé par l'oxygène, le soufre ou l'azote, l'azote étant éventuellement substitué par de l'hydrogène, un radical alkyle (1 ou 2 atomes de carbone), phényle ou benzyle et ce noyau tétragonal à heptagonal pouvant être substitué par 1 à 4 substituants identiques ou différents chosis dans le groupe comprenant le fluor, le chlore, les substituants trifluorométhyle, alkyle, hydroxyalkyle, alkoxyalkyle ou alkoxycarbonyle ayant dans chaque cas 1 à 4 atomes de carbone dans les restes alkyle et alkoxy, ou bien
ce noyau peut être condensé avec un noyau aromatique éventuellement substitué par du fluor, du chlore, un substituant nitro ou hydroxy,

c) $R^7$ possède la définition indiquée en a) pour $R^6$, $R^7$ et $R^6$ pouvant être identiques ou différents, ou bien

d) l'un des deux restes $R^6$ ou $R^7$ représente le groupe

$-N(R^{6'})(R^{7'})$

les restes $R^{6'}$ et $R^{7'}$ ayant la définition indiquée en a) et

b) pour $R^6$ et $R^7$ (hydrazines)

et leurs sels d'addition d'acides pharmaceutiquement acceptables ainsi que leurs formes stéréoisomériques.

3. Composé de formule

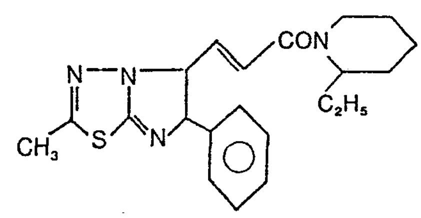

4. Composé de formule

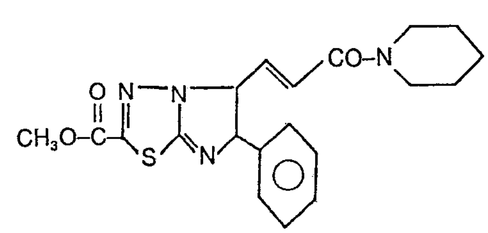

5. Procédé de production de composés de formule générale (I) suivant la revendication 1, caractérisé en ce que

a) on fait réagir des composés carbonyliques de formule générale (II)

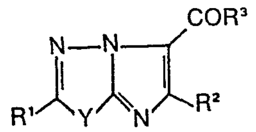

(II)

dans laquelle

X, Y, $R^1$, $R^2$ et $R^3$ ont la définition indiquée dans la revendication 1,

avec des phosphonates de formule générale (III)

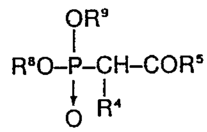

(III)

dans laquelle

$R^4$ et $R^5$ ont la définition indiquée dans la revendication 1, et

$R^8$ et $R^9$ représentent un groupe alkyle ou aralkyle éventuellement substitué,

en présence de bases fortes et de solvants organiques inertes à des températures comprises entre $-20$ et $+40\,°C$, ou bien

b) on fait réagir des composés carbonyliques de formule générale (II) avec des dérivés d'acétamide de formule générale (IV)

$$R^4\text{–}CH_2\text{–}COR^5 \qquad (IV)$$

dans laquelle

$R^4$ et $R^5$ ont la définition indiquée dans la revendication 1, en présence de catalyseurs acides ou basiques et, le cas échéant en présence de solvants organiques intertes à des températures comprises entre 20 et 200 °C, ou bien

c) on forme les amides des acides alcénoïques de formule générale (V)

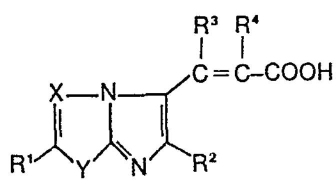

(V)

dans laquelle

X, Y, $R^1$, $R^2$, $R^3$ et $R^4$ ont la définition indiquée dans la revendication 1,

avec des amines de formule générale (VI)

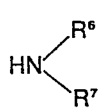

(VI)

dans laquelle

$R^6$ et $R^7$ ont la définition indiquée ci-dessus, éventuellement après activation du groupe carboxyle en passant pas l'halogénure d'acide correspondant, de manière classique, le cas échéant en présence de solvants organiques inertes, à des températures de 20 à 150 °C.

6. Acides alcénoïques de formule générale (V)

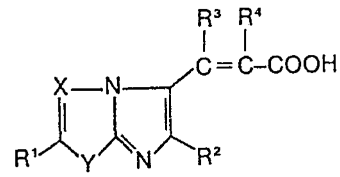

(V)

dans laquelle

X, Y, $R^1$, $R^2$, $R^3$ et $R^4$ ont la définition indiquée dans la revendication 1.

7. Médicament contenant au moins un composé suivant les revendications 1 à 4.

8. Composés suivant les revendications 1 à 4, destinés à être utilisés pour combattre des maladies.

9. Procédé de production d'acides alcénoïques de formule générale (V)

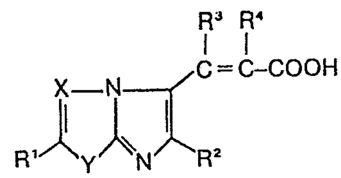

(V)

dans laquelle

X, Y, $R^1$, $R^2$, $R^3$ et $R^4$ ont la définition donnée dans la revendication 1,

caractérisé en ce que

a) on fait réagir des composés carbonyliques de formule générale (II)

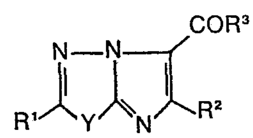

(II)

dans laquelle

X, Y, $R^1$, $R^2$ et $R^3$ ont la définition indiquée ci-dessus, avec des phosphonates de formule générale (VII)

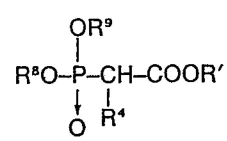

(VII)

dans laquelle

$R^8$, $R^4$ et $R^9$ ont la définition indiquée dans la revendication 5 et

R′ est un groupe alkyle ou aralkyle éventuellement substitué,

pour former des esters d'acides alcénoïques que l'on saponifie ensuite en présence de bases en les acides alcénoïques correspondants de formule générale (V), ou bien

b) au cas où $R^3$ désigne l'hydrogène, on condense des aldéhydes de formule générale (VIII)

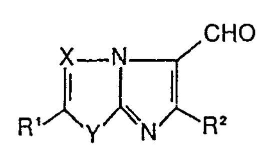

(VIII)

dans laquelle

X, Y, $R^1$ et $R^2$ ont la définition indiquée ci-dessus, avec des acides maloniques de formule générale (IX)

$$\begin{array}{c} HOOC{-}CH{-}COOH \\ | \\ R^4 \end{array} \qquad (IX)$$

dans laquelle

$R^4$ a la définition indiquée ci-dessus,

en présence de solvants organiques inertes et, le cas échéant, en présence d'agents de condensation.